(19) Europäisches Patentamt
European Patent Office
Office européen des brevets

(11) Veröffentlichungsnummer: **0 217 207 B1**

# (12) EUROPÄISCHE PATENTSCHRIFT

(45) Veröffentlichungstag der Patentschrift: **10.04.91**

(51) Int. Cl.$^5$: **C07D 473/00**, A61K 31/52

(21) Anmeldenummer: **86112688.6**

(22) Anmeldetag: **13.09.86**

Teilanmeldung 90113147.4 eingereicht am 13/09/86.

Die Akte enthält technische Angaben, die nach dem Eingang der Anmeldung eingereicht wurden und die nicht in dieser Patentschrift enthalten sind.

## (54) In 6- und 9-Stellung substituierte 2-Aminopurine, ihre Verwendung, diese Purine enthaltende Arzneimittel und Verfahren zur Herstellung der Purine.

(30) Priorität: **24.09.85 DE 3533983**
**02.05.86 DE 3614902**
**09.08.86 DE 3627024**

(43) Veröffentlichungstag der Anmeldung:
**08.04.87 Patentblatt 87/15**

(45) Bekanntmachung des Hinweises auf die Patenterteilung:
**10.04.91 Patentblatt 91/15**

(84) Benannte Vertragsstaaten:
**AT BE CH DE FR GB IT LI LU NL SE**

(56) Entgegenhaltungen:
**EP-A- 0 085 424**
**EP-A- 0 145 207**
**EP-A- 0 152 965**
**CH-A- 629 805**

**JOURNAL OF MEDICINAL CHEMISTRY, Band 28, Nr. 3, März 1985 J.C. MARTIN et al.: "Acyclid Analogues of 2'-Deoxynucleosides Related to 9-(1,3-Dihydroxy-2-propoxy)methyl guanine as Potential Antiviral Agents" Seiten 358-362**

(73) Patentinhaber: **HOECHST AKTIENGESELLSCHAFT**
**Postfach 80 03 20**
**W-6230 Frankfurt am Main 80(DE)**

(72) Erfinder: **Winkelmann, Erhardt, Dr.**
**Brunhildenweg 5**
**W-6233 Kelkheim(DE)**
Erfinder: **Rolly, Heinrich, Dr.**
**Am Tulpengarten 18**
**W-6233 Kelkheim(DE)**
Erfinder: **Winkler, Irvin, Dr.**
**In den Eichen 40**
**W-6237 Liederbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Es sind Arzneimittel bekannt, die 2-Amino-6-hydroxy-9-(2-hydroxyäthoxymethyl)purin (Acyclovir - vgl. Figur 1, Formel ACV in der tautomeren Oxoschreibweise) als Wirkstoff enthalten. Diese stellen gute Antiherpes-Mittel dar (vgl. EP-OS 72 027).

Später sind Arzneimittel gegen virale Infektionen entwickelt worden, die sich von denen der Formel ACV dadurch unterscheiden, daß sie in Nachbarstellung zu der Äthergruppe im Hydroxyäthylrest noch zusätzlich eine Hydroxymethylgruppe enthalten, die also ein Glycerinderivat darstellen, wobei der Sauerstoff in 6-Stellung auch zusätzlich durch Schwefel ersetzt sein kann. (vgl. J. Med. Chem. 28 ,358 (1985)). Derartige Verbindungen sind schließlich weiter dahingehend modifiziert worden, daß die Hydroxymethylgruppe noch durch eine Benzylgruppe veräthert worden ist und gegebenenfalls der Sauerstoff in 6-Stellung durch eine Iminogruppe ersetzt ist (vgl. EP-OS 152 965).

Gegenstand der Erfindung sind nun Verbindungen der Formel 1 (s. Patentanspruch 1) und Arzneimittel, die sie als Wirkstoff enthalten, in denen $R^1$ Wasserstoff, Halogen, Hydroxy, Mercapto, Azido, Amino oder Z-$R^5$ ist, wobei Z Sauerstoff, Schwefel, Sulfinyl, Sulfonyl oder NH und $R^5$ einen acyclischen aliphatischen Kohlenwasserstoffrest mit 1 bis 6 C-Atomen, welcher a) unsubstituiert ist oder b) durch wenigstens eines der Merkmale modifiziert ist, daß er eine Alkoxygruppe mit 1 bis 3 C-Atomen oder eine Doppelbindung enthält oder mindestens teilweise fluoriert ist, oder Cycloalkyl mit 3 bis 6 C-Atomen oder Phenylalkyl mit einer Alkylengruppe von 1 bis 3 C-Atomen oder Phenyl darstellt, wobei der phenylring jeweils höchstens zweifach, vorzugsweise höchstens einmal durch einen Substituenten aus der Gruppe Halogen, Trifluormethyl, Alk oxy und Alkylthio mit jeweils 1 bis 3 C-Atomen substituiert sein kann, $R^2$ Wasserstoff ist oder die Bedeutung von $R^5$ hat oder eine Acylgruppe CO-$R^6$ ist, wobei $R^6$ Alkyl mit 1 bis 6 C-Atomen, Benzyl oder Phenyl ist, $R^3$ Halogen, Azido, unsubstituiertes Amino oder den Rest Y-$R^4$ bedeutet, wobei X und Y Sauerstoff, Schwefel, Sulfinyl oder Sulfonyl bedeuten und gleich oder verschieden sein können und $R^4$ dieselbe Bedeutung wie $R^5$ hat, wobei 9-(1,3-Bis-(isopropoxy)-propyl-2-oxymethyl-guanin und solche Verbindungen ausgenommen sind, in denen nebeneinander $R^1$ OH oder SH $R^2$ H, $R^3$ $YR^4$, X O oder S, Y O und $R^4$ Alkyl mit 1 bis 6 C-Atomen sind bzw. in denen nebeneinander $R^1$ OH oder $NH_2$, $R^2$ Benzyl $R^3$ Y-$R^4$ mit Y=O und $R^4$=Benzyl und X=O ist.

In den Resten $R^4$ bis $R^6$ enthält der acyclische Kohlenwasserstoffrest vorzugsweise 3 bis 5 C-Atome und der cycloaliphatische Rest meistens 4 bis 6 und insbesondere 5 bis 6 C-Atome. Natürlich können die Reste $R^4$ bis $R^6$, in den Phenylresten enthaltene Substituenten, die Reste $R^1$ und $R^3$ sowie X und Y jeweils gleich oder verschieden sein. Aliphatische Kohlenwasserstoffreste, Alkoxyreste und Alkylengruppen in den Phenylalkylresten können jeweils geradkettig oder verzweigt sein, jedoch sind diese in den Phenylalkylresten bevorzugt geradkettig. Halogen bedeutet in den Resten $R^1$ und $R^3$ Fluor, Chlor, Brom oder Jod. Bevorzugt ist Halogen im Rest $R^1$ Chlor oder Brom und im Rest $R^3$ Fluor oder Chlor. Im Rest $R^3$ haben die mindestens teilweise fluorierten Alkylreste z.B. 1 bis 4, Vorzugsweise zwei C-Atome, wobei die teilweise fluorierten Reste besonders bevorzugt sind, beispielsweise $-CH_2-CF_3$.

Die Verbindungen , in denen $R^1$ OH oder SH, $R^2$ H, $R^3$ Y-$R^4$, X O oder S, Y O und $R^4$ Alkyl mit 1 bis 8 C-Atomen darstellen, sind aus der EP-OS 145 207 bekannt und auch ihre Verwendung als Arzneimittel für die Behandlung von autoimmunen Krankheiten wie Arthritis, multipler Sklerose usw. sowie von viralen Infektionen und Krebs. Neben diesen Verbindungen werden in der genannten EP-OS 145 207 auch noch, und zwar hauptsächlich, solche beschrieben und beansprucht, die in 8-Stellung ein Bromatom oder eine $NH_2$- bzw. $NHCOR^6$ -Gruppe enthalten, wobei $R^6$ einen $C_1$-$C_4$-Alkylrest, einen Arylrest oder einen Aralkylrest darstellen kann. Den Verbindungen, die in 8-Stellung Wasserstoff enthalten, werden zwar ebenfalls die obengenannten Wirkungen zugeschrieben, jedoch werden sie vor allem als Zwischenprodukte zur Herstellung der in 8-Stellung substituierten Verbindungen beschrieben und benötigt (vgl. Schema 1, Reaktionsschritte 22(2),23(2), 24(3), 25(4) und 11 (5) sowie Schema 2, Reaktionsschritte 34(2), 33(2), 36(3), 37(4) und 26(5)). Pharmakologische Daten bezüglich der Wirkungsweise werden in der EP-OS 145 207 weder für die in 8-Stellung substituierten Verbindungen noch für die in 8-Stellung unsubstituierten Verbindungen angegeben.

Nach dem Schema 1 der EP-OS 145 207, Reaktionsschritt 22(2) zu 23(2), wird der Benzylrest als Schutzgruppe durch katalytische Hydrierung abgespalten. Derartige Reaktionen sind mit einer Alkylgruppe, wie Isopropylgruppe nicht möglich, weil unter den dort beschriebenen Reaktionsbedingungen zwar die Benzylgruppe als Toluol abgespalten werden kann, nicht aber aliphatische Äthergruppen.

Gegenüber den aus der EP-OS 145 207 bekannten, in 8-Stellung substituierten und in 8-Stellung unsubstituierten Verbindungen und auch gegenüber der Verbindung der Formel Z haben die erfindungsgemäßen Verbindungen den Vorteil der erheblich größeren Wirksamkeit. Dies bedeutet, daß bei gleicher bzw. verbesserter Toxizität wesentlich geringere Mengen der Wirkstoffe verwendet werden können.

Die erfindungsgemäßen Verbindungen der allgemeinen Formel 1 können nach verschiedenen Methoden hergestellt werden, wobei man je nach Bedeutung des Restes $R^1$ verschiedene Ausgangsstoffe und geeignete Schutzgruppen wählt. Einige Ausfüh rungsformen (A bis D) und die im folgenden angegebenen Formelbezeichnungen sind im Reaktionsschema (Figur 2) wiedergegeben, worin HMDS Hexamethyldisilazan, Pyr Pyridin und Ac Acetyl bedeutet und bei den Formeln, in denen $R^1$ OH, SH oder $NH_2$ bedeutet, auch eine Schreibweise in tautomerer Form gemäß Formeln 2', 3' und 6' (s. Figur 1) möglich wäre. In den Fällen, in denen mit Halogenverbindungen umgesetzt wird, ist das Halogen bevorzugt Chlor, wenngleich es auch ein anderes, wie Brom, sein kann.

Die als Ausgangsstoff verwendeten Purine sind bekannt und überwiegend käuflich, so z.B.: 2-Amino-6-hydroxy-purin = Guanin, 2-Amino-6-mercapto-purin und 2-Amino-6-chlor-purin (beide in J.Med.Chem. 28 (3), 358 (1985)), 2,6-Diamino-purin (Synthesis 11 , 963 (1984), EP-OS 138 683) und 2-Amino-purin (EP-OS 108 285). Die Ausgangsstoffe der Formeln 13 und 14 sind überwiegend bekannt und nach bekannten Methoden herstellbar (siehe Beispiele 1a-d und 3a-g).

Verfahren A) Wenn $R^1$ Hydroxy (bzw. in der tautomeren Form Oxo) (Formel 2), Amino (bzw. Imino) (Formel 6), Wasserstoff (Formel 7) oder $R^1 = Z\text{-}R^5$ (Formel 21 ) - s. jeweils Figur 2- ist, setzt man zweckmäßig 2,6,9-Tris-(trimethylsilyl)-bzw. 2,9-Bis-(trimethylsilyl)-2-amino-purine gemäß einer der Formeln 8, 11 und 12 mit Halogen-, insbesondere Chlorverbindungen der Formel 13 um, worin $R^2$ = Acyl ist und X und $R^3$ die angegebene Bedeutung haben, spaltet die Trimethylsilyl-Schutzgruppen ab und erhält so in 9-Position substituierte Verbindungen der Formel 1, speziell der Formeln 15, 19, 20 und 22. Dabei zeichnet sich in Formel 13 das in α-Stellung zu X stehende Halogen, insbesondere Chlor durch eine besondere Reaktivität aus. Natürlich kann man anstelle der Trimethylsilylschutzgruppe auch andere Trialkylsilylschutzgruppen verwenden, in denen der Alkylrest z.B. 1 bis 6, vorzugsweise 1 bis 3 C-Atome hat und geradkettig oder verzweigt ist.

Die erfindungsgemäßen Verbindungen 15, 19 und 20 werden gegebenenfalls zu anderen Verbindungen der Formel 1, in denen $R^2$ statt Acyl Wasserstoff ist, verseift (s. Figur 2).

Verfahren B) Wenn $R^1$ Hydroxy (bzw. Oxo) (Formel 9), Mercapto (bzw. Thio) (Formel 10), Amino (bzw. Imino) (Formel 6a) oder Wasserstoff (Formel 7a) - s. jeweils Figur 1 ist, setzt man entsprechende Di- bzw. Triacyl-Verbindungen der Formeln 9, 10, 6a bzw. 7a mit reaktiven Acetoxy-Verbindungen der Formel 14 um, worin $R^2$ Acyl ist und X und $R^3$ die angegebene Bedeutung haben. Man erhält so in 9-Position substituierte Verbindungen der Formeln 16, 17, 6b und 7b. Diese erfindungsgemäßen Verbindungen werden gegebenenfalls zu anderen Verbindungen der Formel 1 verseift, in denen $R^2$ Wasserstoff ist.

Verfahren C) Wenn $R^1$ Halogen, insbesondere Chlor ist (Formel 5), kann man $C_1$) diese Verbindung mit aktiven Halogenverbindungen der Formel 13 zu in 9-Position substituierten Verbindungen der Formel 18 umsetzen und diese $c_2$) gegebenenfalls zu anderen Verbindungen der Formel 1, in denen $R^2$ H ist, verseifen, oder $C_3$) vor oder nach der Verseifung zur Herstellung anderer Verbindungen der Formel 1 das Halogen austauschen, z.B. wenn

- $R^1$ Hydroxy (bzw. Oxo) sein soll, durch saure Verseifung, z.B. mit wäßriger Salzsäure,
- $R^1$ Mercapto (bzw. Thio) sein soll, durch Umsetzung mit Schwefelwasserstoff,
- R [1] Azido sein soll, durch Umsetzung mit Alkaliaziden,
- $R^1$ Amino (bzw. Imino) sein soll, durch Umsetzung mit Ammoniak,
- $R^1$ Wasserstoff sein soll, durch katalytische dehalogenierende Hydrierung, z.B. mit Palladium-Kohle/Wasserstoff + Triäthylamin,
- $R^1$ $ZR^5$ sein soll, durch Umsetzung mit Alkoholen bzw. Phenolen, Mercaptanen bzw. Thiophenolen oder Aminen bzw. Anilinen,

wobei $R^2$ Acyl oder gegebenenfalls nach Verseifung Wasserstoff ist und $R^3$ und X die angegebene Bedeutung haben.

Bevorzugt für das Verfahren C sind vielfach Verbindungen, in denen $R^1$ H, OH oder $NH_2$, $R^2$ Acyl, insbesondere Acetyl, und X O oder S sind, wobei $R^3$ und damit auch Y und $R^4$ die im Anspruch 1 angegebene Bedeutung haben.

Die Umsetzungen nach den Verfahren A bis C und dem weiter unten genannten Verfahren D, die Analogieverfahren darstellen, werden unter den üblichen Bedingungen durchgeführt. Bei den Verfahren A) bis c) liegen die Reaktionstemperaturen im allgemeinen zwischen 0 und 150° C, vorzugsweise zwischen 50 und 100° C. Gewöhnlich arbeitet man bei Normaldruck, jedoch ist es auch möglich, bei erhöhtem oder vermindertem Druck zu arbeiten, wenngleich hiermit normalerweise keine Vorteile verbunden sind. Die Lösungs- und Verteilungsmittel können natürlich sowohl in Form einheitlicher Stoffe als auch in Form von Gemischen verwendet werden. Zu den einzelnen Verfahren wird noch folgendes ausgeführt:

Die Umsetzungen nach Verfahren A) werden zweckmäßig in einem Lösungs- oder Verteilungsmittel durchgeführt. Dafür kommen insbesondere aprotische Lösungsmittel in Frage, wie Benzol, Toluol, die

verschiedenen Xylole, ferner 1,2-Dichloräthan, Chlorbenzol, 1,2-Dimethoxyäthan, Dioxan. Zum Abfangen der gebildeten Halogenwasserstoffsäure empfiehlt sich die Verwendung einer Base, wie Triäthylamin oder N-Äthylmorpholin. Die Reaktionszeiten betragen je nach Reaktionstemperatur und Reaktionspartner einige Minuten bis mehrere Stunden, meistens 12-24 Stunden.

Wegen der Feuchtigkeitsempfindlichkeit der als Ausgangsstoffe verwendeten trialkylsilyl-geschützten Purine 8, 11, 12 empfiehlt sich die Verwendung eines Schutzgases im Reaktionsgefäß, z.B. Stickstoff oder Argon. Die Abspaltung der Trialkylsilyl-Schutzgruppen kann z.B. durch Kochen der Zwischenverbindung mit einem Alkohol, wie Methanol oder Äthanol erfolgen.

Die Umsetzungen nach Verfahren B) werden zweckmäßig ebenfalls in einem Lösungs- oder Verteilungsmittel durchgeführt. Dafür kommen insbesondere Sulfone als aprotische Lösungsmittel in Frage, wie Sulfolan (Tetramethylensulfon) oder Dipropylsulfon. Bei dieser Umsetzung empfiehlt sich die Verwendung eines sauren Katalysators, wie Methan-, Äthan-, Benzol- oder Toluol-sulfonsäure. Die Reaktionszeiten betragen je nach Reaktionstemperatur und Reaktionspartner meist mehrere Stunden bis zu einigen Tagen.

Die Umsetzungen nach Verfahren $C_1$ werden ebenfalls zweckmäßig in einem Lösungs- oder Verteilungsmittel durchgeführt. Dafür kommen insbesondere polare, aprotische Lösungsmittel in Frage, wie Dimethylformamid, Dimethylacetamid, Tetramethylharnstoff, Hexamethylphosphorsäuretriamid oder Dimethylsulfoxid. Zum Abfangen der gebildeten Halogenwasserstoffsäure empfiehlt sich die Verwendung einer Base, z.B. der weiter unten bei der Verseifung genannten, wie Triäthylamin, N-Äthylmorpholin oder Alkalicarbonate, wie Natrium- oder Kaliumcarbonat. Die Reaktionszeiten betragen je nach Reaktionstemperatur und Reaktionspartner mehrere Minuten bis mehrere Stunden.

Der Halogenaustausch nach Verfahren $C_2$ wird z.B. auf folgende Weise durchgeführt:

$C_2a$) Wenn $R^1$ Hydroxy (bzw. Oxo) ist, kann die Verseifung der Verbindung 18, z.B mit 1-molarer wäßriger Salzsäure, durch 2-3-stündiges Kochen unter Rückfluß zur Verbindung 15 erfolgen; derartige Reaktionsbedingungen sind, ebenso wie die für das Verfahren $C_1$), eingehend in der Internationalen Anmeldung PCT/SE83/00254 beschrieben.

$C_2b$) Wenn $R^1$ Mercapto (bzw. Thio) ist, kann die Umsetzung der Verbindung 18 mit Schwefelwasserstoffgas in Gegenwart einer Base, wie Triäthylamin oder Pyridin, die gleichzeitig als Lösungsmittel dienen kann, oder in einem Alkohol wie Methanol, Äthanol, einem der Propanole oder Butanole, Äthylenglykolmonomethyläther oder -äthyläther, z.B. bei 20-60 °C, zur Verbindung 17, erfolgen.

$C_2c$) Wenn $R^1$ Azido ist, kann die Umsetzung mit Alkaliaziden in polaren, eventuell auch in unpolaren, gegenüber den Reaktionsteilnehmern inerten Lösungsmitteln wie Dimethylform-bzw. -acetamid, Dimethylsulfoxyd, Hexamethylphosphorsäuretriamid, N-Methylpyrrolidon, Acetonitril und Tetramethylharnstoff unter üblichen Bedingungen, z. B. zwischen 20 und 120, vorzugsweise zwischen 60 und 90 °C erfolgen.

$C_2d$) Wenn $R^1$ Amino (bzw. Imino) ist, kann die Umsetzung der Verbindung 18 z.B. mit Ammoniak (flüssig oder in alkoholischer Lösung), gegebenenfalls unter Verwendung eines gegenüber den Reaktionsteilnehmern inerten Lösungsmittels, wie Äthanol, im allgemeinen bei Temperaturen zwischen 20 und 120 °C, vorzugsweise 50-100 °C erfolgen, z.B. innerhalb von 12 Stunden.

$C_2e$) Wenn $R^1$ Wasserstoff ist, kann die dehalogenierende Hydrierung der Verbindung 18 vorteilhaft mit Palladium-Kohle/Wasserstoff unter atmosphärischem Druck in Gegenwart einer hierfür geeigneten Base, wie Triäthylamin, unter üblichen Bedingungen, z.B. in 12 Stunden bei Raumtemperatur, zur Verbindung 20 durchgeführt werden.

$C_2f$) Wenn $R^1$ Z-$R^5$, Z Sauerstoff, Schwefel oder NH und $R^5$ die angegebene Bedeutung haben, kann die Umsetzung der Verbindung 18 mit entsprechenden Hydroxy-, Mercapto- oder Aminoverbindungen erfolgen. Die Hydroxy- oder Mercaptoverbindungen werden in Form ihrer Alkalisalze, die Aminoverbindungen in Gegenwart einer anorganischen oder vorzugweise organischen Base, wie Triäthylamin oder Pyridin, zur Umsetzung gebracht. Als Lösungsmittel kann bei den Hydroxyverbindungen eingesetzter Alkohol im Überschuß, bei Aminoverbindungen z.B. die Hilfsbase Pyridin verwendet werden. Im allgemeinen verwendet man als Lösungsmittel Alkohole, wie Propanole und Butanole, Alkylenglykole oder ihre Mono- bzw. Dialkyläther. Die Temperaturen liegen im allgemeinen zwischen 25 und 130 °C, vorzugsweise zwischen 80 und 100 °c. Die Reaktionszeiten liegen je nach Temperatur und Reaktionsteilnehmer meistens zwischen 6 und 24 Stunden. Durch analoge Umsetzung der Verbindung 3 bzw. 5 mit entsprechenden Hydroxy-, Mercapto- oder Aminoverbindungen und weitere Umsetzung mit Verbindung 13 bzw. 1 nach Verfahren A) lassen sich Verbindungen mit $R^1$ = Z-$R^5$ (Verbindung 21 bzw. 22) herstellen, wie in Figur 2 dargestellt.

Verfahren D) Soweit nach einem der Verfahren A bis C . erfindungsgemäße Verbindungen wie 15 bis 20, 6b und 7b erhalten werden, können diese, z.B. durch Einwirkung von Basen, wie Ammoniak, Alkylaminen, wie Methyl- oder Äthylamin, den verschiedenen Propyl- oder Butylaminen, oder Alkalihydroxyden oder -carbonaten, wie Natriumhydroxid, Kaliumhydroxid, Natriumcarbonat, Kaliumcarbonat in wäßriger

oder alkoholischer (Methanol, Äthanol) Lösung, im allgemeinen bei Temperaturen von 0-100 °C, vorzugsweise bei 20-50 °C verseift werden.

Die meisten Verbindungen der Formeln 15 bis 19 fallen nach Verfahren A bis D als Gemisch von Purin-7- und -9-Isomeren in wechselnden Mengenverhältnissen an, die gegebenenfalls durch fraktionierte Kristallisation getrennt werden können. Im allgemeinen wird die Isomerentrennung mittels Säulenchromatographie z.B. über Kieselgel durchgeführt. Die Charakterisierung der Isomeren und ihre Prozentgehaltbestimmung erfolgt z.B. durch Hochdruckflüssigkeitschromatographie (HPLC) unter Verwendung von (R) Li-Chrosorb Si 60/5 µm der Firma Merck, Darmstadt, Deutschland.

Die erfindungsgemäßen Verbindungen besitzen, soweit nicht Y Sauerstoff und $R^2$ mit $R^4$ identisch ist, ein optisch aktives (chirales) Zentrum am Kohlenstoff-2-atom in der Ätherseitenkette. Die Verbindungen liegen als Racemate (DL-Form) vor. Eine Herstellung bzw. Isolierung der optisch aktiven Antipoden (Enantiomeren) ist möglich.

Die erfindungsgemäßen Verbindungen der Formel 1 besitzen wertvolle pharmazeutische Eigenschaften. Sie sind in vitro und in vivo gegen verschiedene Viren wirksam und eignen sich daher zur Bekämpfung verschiedener, durch DNS-Viren verursachter Krankheiten, wie Herpes, z. B. Typus simplex 1 und 2, Cytomegalie und RNS-Viren, z. B. Retroviren, sowie Autoimmunkrankheiten und zur Behandlung von Krebs. Arzneimittel, die sie enthalten, können enteral (oral), parenteral (intravenös), rektal oder lokal (topisch) angewendet werden. Sie können in Form von Lösungen, Pulvern (Tabletten, Kapseln einschließlich Mikrokapseln), Salben (Cremes oder Gel) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0,1-10, vorzugsweise 0,2-8 mg/kg Körpergewicht verabreicht. Sie werden zweckmäßig in Dosierungseinheiten verabreicht, die mindestens die wirksame Tagesmenge der erfindungsgemäßen Verbindungen, z.B. 30-300, vorzugsweise 50-250 mg enthalten.

Die erfindungsgemäßen Verbindungen können auch in Kombination mit anderen Antivirusmitteln und Immunstimulantien, wie Interferonen verabreicht werden.

Die Wirkung erfindungsgemäßer Verbindungen wurde wie folgt geprüft:

A. In vitro-Versuche:

Zellkulturen, z.B. Hela- und Vero-Zellen, wurden in Mikrotiterplatten eingesät und mit Viren, z.B. Herpes simplex 1 infiziert. 2 Stunden nach der Infektion wurden die zu prüfenden Verbindungen den infizierten Zellkulturen in verschiedenen Verdünnungen zugegeben. 48-72 Stunden nach der Infektion wurde der Therapieerfolg anhand des cytopathogenen Effektes mikroskopisch und nach Neutralrotaufnahmen photometrisch bestimmt.

B. In vivo-Versuche:

NMRI-Mäuse, spezifisch pathogenfrei, im Gewicht von etwa 15 g wurden intraperitoneal mit Herpes simplex Typ 1 infiziert und anschließend mit den beschriebenen Verbindungen intraperitoneal therapiert. Als Vergleich diente stets Acyclovir (ACV). Die Behandlung erfolgte zweimal täglich über 5 Tage, beginnend nach der Infektion. Der Behandlungserfolg wurde anhand des Krankheitsverlaufes und der Überlebensrate gegenüber den unbehandelten Infektionskontrollen bestimmt. Letztere erhielten anstelle der zu prüfenden Verbindungen eine wasserlösliche Methylhydroxyäthylcellulose (Viskosität 300 Pa•s in 2 %iger Lösung) ((R)Tylose MH 300 P) appliziert.

Die chemotherapeutische Wirkung erfindungsgemäßer Verbindungen ist aus Tabelle 1 ersichtlich.

## Beispiele

1) Erfindungsgemäße Produkte:

1a) 9-(1-Clormethyl-2-acetoxyäthoxymethyl)-guanin (Verbindung 6)

36,73 g (0,1 Mol) 2,6,9-Tris-(trimethylsilyl)-guanin, Rohprodukt (hergestellt nach US-Patent 4.287.188), werden in 100 ml Toluol gelöst, 20 ml Triäthylamin zugegeben und 22,0 g (0,1 Mol) + 10 % Überschuß)

1-Chlor-2-chlormethoxy-3-acetoxy-propan, gelöst in etwas Toluol, bei Raumtemperatur unter Rühren und unter Stickstoff-Schutzgas zugetropft. Die Reaktionsmischung wird sodann 20 Stunden unter Rühren und unter Stickstoff auf 90° C erhitzt. Danach wird das Lösungsmittel Toluol abgedampft, der Rückstand in 500 ml Methanol gelöst und 30 Minuten unter Rückfluß erhitzt. Die Lösung wird nach Kohlezusatz warm abgesaugt, eingedampft und der Rückstand zweimal aus Wasser umkristallisiert. Man erhält 16 g = 50 % d. Theorie; weiße Kristalle; Fp. 166° C.

1b) 9-(1-Chlormethyl-2-hydroxy äthoxymethyl)-guanin (Verbindung 2)

31,55 g (0,1 Mol) der nach 1a erhaltenen Verbindung werden in eine Lösung von 500 ml flüssigem Ammonaik in 500 ml Methanol eingetragen. Die Lösung wird 12 Stunden bei Raumtemperatur gerührt, nach Zusatz von etwas Aktivkohle abgesaugt, eingedsmpft und der Rückstand zweimal aus wenig Wasser umkristallisiert. Man erhält 19 g = 70 % d. Theorie; weiße Kristalle; Fp. 178° C.

Ausgangsstoffe

1c) 1-Chlor-2-chlormethoxy-3-acetoxy-Propan

15,25 g (0,1 Mol ) 1-Chlor-2-hydroxy-3-acetoxy-propan werden in 70 ml Methylenchlorid gelöst, 3 g (0,1 Mol) Paraformaldehyd zugegeben und unter Rühren und Eis/Wasser-Kühlung bei ca. 5° C 2 Stunden lang Chlorwasserstoffgas eingeleitet. Nach Zugabe von 10 g Magnesiumsulfat (wasserfrei) als wasserbindendem Mittel wird nochmals 4 Stunden bei 5° C Chlorwasserstoffgas eingeleitet. Sodann wird das Magnesiumsulfat abgesaugt und das Lösungsmittel bei möglichst niedriger Temperatur abgedampft. Der flüssige Rückstand wird direkt weiter umgesetzt. Man erhält 17 g= 85 % d. Theorie; Flüssigkeit; zersetzlich bei der Destillation.

1d) 1-Chlor-2-hydroxy-3-acetoxy-propan

Die als Ausgangsstoff verwendete Verbindung wurde nach Liebig's Annalen 402, 137 (1914) durch Umsetzung von Epichlorhydrin mit Eisessig in Gegenwart von Eisen-(III)-chlorid bei Raumtemperatur dargestellt. Flüssigkeit; Kp. 17 mm 118° C.

2) Erfindunsgemäße Produkte

2a)9-(1-Azidomethyl-2-acetoxyäthoxymethyl)-guanin (Verbindung 9)

36,73 g (0,1 Mol) 2,6,9-Tris-(trimethylsilyl)-guanin, Rohprodukt werden in 100 ml Toluol gelöst, 20 ml Triäthylamin zugegeben und 22,8 (0,1 Mol + 10 % Überschuß) 1-Azido-2-chlormethoxy-3-acetoxypropan, gelöst in etwas Toluol, zunächst bei Raumtemperatur ,unter Rühren und unter Stickstoff-Schutzgas zugetropft. Die Reaktionsmischung wird sodann 20 Stunden unter Rühren und unter Stickstoff auf 80° C erhitzt. Danach wird das Toluol abgedampft, der Rückstand in 500 ml Methanol gelöst und 30 Minuten unter Rückfluß erhitzt. Die Lösung wird nach Kohlezusatz warm abgesaugt, eingedampft, der Rückstand in einer Mischung von Methylenchlorid/Methanol 5:1 (Volumenverhältnis) gelöst und über eine Chromatographie-Säule, beladen mit Kieselgel (Grace Matrex L C 60 A) gereinigt. Das Eluat wird eingedampft, der Rückstand mit Äthanol angerührt, abgesaugt und mit wenig Äthanol und Äther gewaschen. Man erhält 13,7 g = 38 % der Theorie; weiße Kristalle, Fp. 198° C.

2b) 9-(1-Aminomethyl-2-acetoxy äthoxymethyl)-guanin (Verbindung 10)

Die nach 2a) erhaltene Verbindung wird katalytisch mit Raney-Nickel/Wasserstoff in Äthylenglykolmonomethyläther bei 40° C reduziert. Weiße Kristalle; Fp. 144° C.

Ausgangsstoffe

2c) 1-Azido-2-chlormethoxy-3-acetoxy-propan

15,9 g (0,1 Mol) 1-Azido-2-hydroxy-3-acetoxy-propan werden in 100 ml Methylenchlorid gelöst, 3 g (0,1 Mol) Paraformaldehyd zugegeben und unter Rühren und Eis/Wasser-Kühlung bei 0-5° C 2 Stunden lang Chlorwasserstoffgas eingeleitet. Nach Zugabe von 10 g Natriumsulfat (wasserfrei) wird weitere 4 Stunden bei 5° C Chlorwasserstoffgas eingeleitet. Sodann wird vom Natriumsulfat abgesaugt und das Lösungsmittel bei möglichst niedriger Temperatur abgedampft. Der flüssige Rückstand wird direkt weiter umgesetzt. Man erhält 1-Azido-2-chlormethoxy-3-acetoxy-propan. Dieses wird als Rohprodukt für die Herstellung der Verbindung 9 gemäß Beispiel 2a verwendet.

2d) 1-Azido-2-hydroxy-3-acetoxy-propan

13,55 g (0,1 Mol) 1-Azido-2-hydroxy-3-chlorpropan werden mit 10,7 g (0,1 Mol + 10 % Überschuß), Kaliumacetat wasserfrei, versetzt und 1 Stunde unter Rühren auf 150° C erhitzt. Nach dem Erkalten wird auf 200 ml Eis/Wasser gegossen und 3 Mal mit Äther ausgeschüttelt. Die Ätherextrakte werden über

Natriumsulfat getrocknet, eingedampft und der Rückstand unter Vakuum der Wasserstrahlpumpe destilliert. Man erhält die Verbindung als gelbliches Öl; $Kp_1$ 80° C.

2e) 1-Azido-2-hydroxy-3-chlor-propan

Diese Verbindung wurde nach J.Org.Chem. 21 , 373 (1956) durch Umsetzung von 12,0 g (0,13 Mol) Epichlorhydrin mit 11,7 g (0,18 Mol) Natriumazid in Gegenwart von 17,9 g (0,08 Mol) Magnesiumperchlorat in Wasser bei 0 bis 5° C hergestellt. Die Verbindung ist eine farblose Flüssigkeit vom $Kp_1$ 62° C.

Erfindungsgemäße Produkte

3a) 9-(1-Isopropoxymethyl-2-acetoxy äthoxymethyl)-guanin (Verbindung 20)

36,73 g (0,1 Mol) 2,6,9-Tris-(trimethylsilyl)-guanin, Rohprodukt (hergestellt nach US-Patent 4.287.188), werden in 100 ml Toluol gelöst, 20 ml Triäthylamin zugegeben und 24,6 g (0,1 Mol + 10 % Überschuß) 1-isopropoxy-2-chlormethoxy-3-acetoxy-propan, gelöst in etwa Toluol, bei Raumtemperatur unter Rühren und unter Stickstoff-Schutzgas zugetropft. Die Reaktionsmischung wird zunächst, wie im Beispiel 2 beschrieben, behandelt und eingedampft. Der eingedampfte Rückstand enthält je nach Ansatz die 7- und 9-Isomeren in wechselnden Mengenverhältnissen. Die Schmelzpunkte schwanken daher zwischen 209 und 225° C. Eine Trennung dieser Isomeren ist über eine Büchi-Chromatographlesäule, gefüllt mit Grace-Kieselgel (R)Matrex LC 60A und der Laufmittelmischung Methylenchlorid/Cyclohexan/Methanol/Eisessig (Volumenverhältnis 100:20:10:1) möglich. Nach Eindampfen des Eluats wird der Rückstand mit Wasser angerührt, abgesaugt und mit Wasser und wenig kaltem Äthanol gewaschen und getrocknet. Aus einem Rückstand vom Fp. 212° C erhält man so 9 g =(26 % d. Theorie), weiße Kristalle vom Fp. 232° C (95 % 9-Isomeres nach HPLC-Analyse Über Merck Li-Chrosorb Si 60/5 µm).

3b)9-(1-Isopropoxymethyl-2-hydroxy äthoxymethyl)-guanin (Verbindung 14)

33,9 g (0,1 Mol) 9-(1-Isopropoxymethyl-2-acetoxyäthoxy methyl)-guanin werden in eine Lösung von 500 ml flüssigem Ammoniak in 500 ml Methanol eingetragen. Die Lösung wird 12 Stunden bei Raumtemperatur gerührt, nach Zusatz von etwas Aktivkohle abgesaugt, eingedampft und der Rückstand 2 mal aus Wasser umkristallisiert. Man erhält 17 g = 57 % der Theorie; weiße Kristalle, Fp. 191° C.

Dieselbe Verbindung 14 kann auch nach Verfahren B wie folge hergestellt werden. Dazu werden 23,5 g (0,1 Mol) 2,9-Diacetyl-guanin zusammen mit 27,3 g (0,1 Mol + 10 % Überschuß) 1-Isopropoxy-2-acetoxy-methoxy-3-acetoxy-propan und 0,2 g 4-Toluolsulfonsäure in 60 ml wasserfreiem Sulfolan 60 Stunden unter Rühren auf 95° C erhitzt. Die erkaltete Reaktionsmischung wird mit 500 ml Toluol verdünnt, filtriert und über Kieselgel chromatographiert. Die Eluation erfolgt mit einer Mischung von Methylenchlorid/Methanol (Volumenverhäntis 10:1). Das Eluat wird eingedampft, der Rückstand (2-Acetyl-7(9)-(1-Isopropoxymethyl-2-acetoxy-äthoxymethyl)-guanin-Isomerengemisch) wird in eine Lösung von 500 ml flüssigem Ammoniak in 500 ml Methanol eingetragen und 12 Stunden bei Raumtemperatur gerührt. Nach Zusatz von etwas Aktivkohle wird abgesaugt, eingedampft und der Rückstand aus Wasser umkristallisiert. Man erhält 9 g = 37 % d. Theorie, weiße Kristalle, Fp. 190° C.

Ausgangsstoffe

3c) 1-Isopropoxy-2-chlormethoxy-3-acetoxy-propan

17,6 g (0,1 Mol) 1-Isopropoxy-2-hydroxy-3-acetoxy-propan werden mit Formaldehyd und Chlorwasserstoffgas analog Beispiel 2c) umgesetzt. Man erhält 20 g der Verbindung als Flüssigkeit (90 % der Theorie); zersetzlich bei der Destillation.

3d) 1-Isopropoxy-2-hydroxy-3-acetoxy-propan

15,25 g (0,1 Mol) 1-Isopropoxy-2-hydroxy-3-chlor-propan werden mit wasserfreiem Kaliumacetat analog Beispiel 2d) umgesetzt. Man erhält 15 g = 85 % der Theorie; Flüssigkeit; $Kp_{22}$ 128° C.

3e) 1-Isopropoxy-2-hydroxy-3-chlor-propan

Die als Ausgangsstoff verwendete Verbindung wurde nach J.Org.Chem. 12 , 831 (1947) durch Umsetzung von Epichlorhydrin mit Isopropanol in Gegenwart von konzentrierter Schwefelsäure bei Raumtemperatur dargestellt. Flüssigkeit; $Kp_{22}$ 89° C.

3f) 2,9-Diacetyl-guanin

Diese Verbindung wurde analog Bull.Chem. Soc.Jap. 37 (9), 1389 durch Umsetzung von Guanin mit Acetanhydrid in Gegenwart von etwas konz. Schwefelsäure (30 Minuten Rückfluß) hergestellt. Fp. 265° C.

3g) 1 Isopropoxy-2-acetoxymethoxy-3-acetoxy-propan

Diese Verbindung wurde durch Umsetzung von 1-Isopropoxy-2-chlormethoxy-3-acetoxy-propan (3c) mit überschüsigem Kaliumacetat in Aceton (12 Stunden bei Raumtemperatur) analog J.Med.Chem. 26 , 759 (1983) hergestellt und als Rohprodukt direkt weiter umgesetzt.

Erfindungsgemäße Produkte

4a) 2-Amino-6-isopropoxy-9-(1-isopropoxymethyl-2-acetoxy-äthoxymethyl)-purin (Verbindung 44)
19,3 g (0,1 Mol) 2-Amino-6-isopropoxy-purin und 13,2 g (0,1 Mol) Ammoniumsulfat werden in 200 ml Hexamethyldisilazan unter Stickstoff eingetragen und 4 Stunden auf 130 °C erhitzt. Nach Abkühlen wird das überschüssige Hexamethyldisilazan unter Wasserstrahlvakuum abdestilliert, der feste Rückstand (6-Isopropyl-2,9-bis-(trimethylsilyl)-guanin) in 100 ml Toluol gelöst, 20 ml Triäthylamin zu gegeben und unter Rühren und unter Stickstoff 24,6 g (0,1 Mol + 10 % Überschuß) 1-Isopropoxy-2-chlormethoxy-3-acetoxy-propan, gelöst in etwas Toluol, bei Raumtemperatur zugetropft. Die Reaktionsmischung wird sodann 12 Stunden unter Rühren und Stickstoff auf 90 °C erhitzt. Danach wird das Toluol abgedampft, der Rückstand in 200 ml Methanol gelöst und 20 Minuten unter Rückfluß erhitzt. Das Methanol wird abgedampft, der Rückstand in Methylenchlorid/Methanol (Volumenverhältnis 40:1) gelöst und über eine Chromatographiesäule, beladen mit Kieselgel (Fa. Grace, Matrex LC 60A gereinigt. Das Eluat wird eingedampft, der ölige Rückstand durch Anrühren mit Essigsäureäthylester/Cyclohexan (Volumenverhältnis 2:1) zur Kristallisation gebracht. Die Kristalle werden abgesaugt und mit Essigsäureäthylester/Diäthyläther gewaschen und im Exsikkator getrocknet. Man erhält 19 8 = 50 % der Theorie; weiße Kristalle; Fp. 137 °C (98 % 9-Isomeres nach HPLC-Analyse über Merck Li-Chrosorb Si 60/5 µm).

Ausgansstoffe

4b) 2-Amino-6-isopropoxy-purin
Diese Verbindung wurde durch Umsetzung von 2-Amino-6-chlor-purin mit Natriumisopropylat in Isopropanol (12-stündiges Kochen unter Rückfluß) analog J.Org.Chem. 25 , 1573 (1960) in hohen Ausbeuten hergestellt. Fp. 206 °C.

Erfindungsgemäße Produkte

5a) 9-(1,3-Bis-(isopropoxy)-propyl-2-oxymethyl)-guanin (bekannt aus der EP-A-187 297) Verbindung 49
36.73 g (0,1 Mol) 2,6,9-Tris-(trimethylsilyl)-guanin, Rohprodukt, werden mit 24,7 g (0,1 Mol + 10 % Überschuß) 1,3-Bis-(isopropoxy)-2-chlormethoxy-propan in Gegenwart von 20 ml Triäthylamin in Toluol wie unter Beispiel 2b) umgesetzt. Danach wird das Toluol abgedampft, der Rückstand in 200 ml Methanol gelöst und 20 Minuten unter Rückfluß erhitzt. Das Methanol wird abgedampft, der Rückstand in Methylenchlorid/Methanol (Volumenverhältnis 9:1) gelöst und über Kieselgel Fa. Grace, Matrex LC 60A chromatographiert. Das Eluat wird eingedampft, der kristalline Rückstand aus Äthanol umkristallisiert. Man erhält 9,5 g = 42% der Theorie; weiße Kristalle; Fp. 222 °C (96 % 9-Isomeres nach HPLC-Analyse über Merck Li-Chrosorb Si 60/5 µm).

Ausgangsstoffe

5b) 1,3-Bis-(isopropoxy)-2-chlormethoxy-propan
17,6 g (0,1 Mol) 1,3-Bis-(isopropoxy)-2-hydroxy-propan wurden mit 3 g (0,1 Mol) Formaldehyd und Chlorwasserstoffgas bei 0 bis 5 °C hergestellt und als Rohprodukt direkt weiter umgesetzt.
5c) 1,3-Bis-(isopropoxy)-2-hydroxy-propan
11,6 g (0,1 Mol) 2,3-Epoxypropyl-isopropyläther wurde mit Isopropanol in Gegenwart von katalytischen Mengen Zinn-IV-chlorid zunächst bei Raumtemperatur und danach bei langsam auf 80 °C ansteigender Temperatur umgesetzt. Farblose Flüssigkeit; Kp 9 mm 80 °C.
6 ff) Nach den unter 1 bis 5) beschriebenen Verfahren lassen sich die in der Tabelle 2 aufgeführten Verbindungen herstellen. Diese Verbindungen sind dort außer durch ihre Schmelzpunkte durch die NMR-Spektren identifiziert. Verbindung 40 ist 2-Amino-9-(1-isopropoxymethyl-2-acetoxyäthoxymethyl)-purin, Verbindung 48 ist das 2-Amino-9-[1,3-bis-(isopropoxy)-propyl-2-oxymethyl]-purin und Verbindung 52 ist das 2-Amino-6-isopropoxy-9-[1,3-bis-(isopropoxy)-propyl-2oxymethyl]-purin.

## Tabelle 1

Herpes simplex Typ 1 an der NMRI-Maus

| Präparat No. | Dosierung mg/Maus i.p. | Mittlere Überlebens-rate x/10 | Dosierung mg/Maus i.p. | Mittlere Überlebens-rate x/10 |
|---|---|---|---|---|
| 6 | 10 x 0,6 | 9/10 | 10 x 0,2 | 8/10 |
| 7 | " | 9/10 | " | 8/10 |
| 9 | " | 9/10 | " | 9/10 |
| 14 | " | 10/10 | " | 8/10 |
| 20 | " | 10/10 | " | 10/10 |
| 22 | " | 10/10 | " | 9/10 |
| 24 | " | 10/10 | " | 8/10 |
| 28 | " | 10/10 | " | 8/10 |
| 29 | " | 10/10 | " | 8/10 |
| 30 | " | 10/10 | " | 8/10 |
| 31 | " | 9/10 | " | 7/10 |
| 39 | " | 10/10 | " | 8/10 |
| 40 | " | 10/10 | " | 8/10 |
| 42 | " | 10/10 | " | 8/10 |
| 44 | " | 10/10 | " | 9/10 |
| 48 | " | 10/10 | " | 9/10 |
| 49 | " | 10/10 | " | 9/10 |
| 52 | " | 10/10 | " | 9/10 |
| Acyclovir | | 9/10 | | 8/10 |
| Infektions-kontrollen | 10 x 0,5 ml Tylose | 2/10 | 10 x 0,5 ml Tylose® | 2/10 |

Acyclovir = 9-(2-Hydroxyäthoxymethyl)-guanin

Tabelle 2

| No. | $R^1$ | $R^2$ | X | $R^3$ | Fp. °C | s, 2H $NH_2$ C-2 | s, 1H C-6 | s, 1H CH C-8 | s, 2H $NCH_2X$ | m, 1H XCH< | m, 2H $CH_2OR^2$ | s, 3H $OCOCH_3$ | m, 2H $CH_2R^3$ | m $R^3$ |
|---|---|---|---|---|---|---|---|---|---|---|---|---|---|---|
| | Formel I | | | | | NMR δ-Werte in Dimethylsulfoxyd ($Me_2SO-d_6$, 270 MHz) | | | | | | | | |
| 1 | OH | H | O | F | 180 | 6,45 | 10,6 | 7,8 | 5,3 | 4,15 | 3,7 | – | 4,1 | |
| 2 | | | | Cl | 178 | 6,45 | 10,6 | 7,8 | 5,3 | 4,15 | 3,7 | – | 4,1 | |
| 3 | | | | Br | 158 | 6,45 | 10,6 | 7,8 | 5,3 | 4,15 | 3,7 | – | 4,1 | |
| 4 | | | | J | 150 | 6,45 | 10,6 | 7,8 | 5,3 | 4,15 | 3,7 | – | 4,1 | |
| 5 | OH | Ac | O | F | 171 | 6,45 | 10,6 | 7,8 | 5,4 | 4,15 | 3,6 | 2,0 | 4,1 | |
| 6 | | | | Cl | 166 | 6,45 | 10,6 | 7,8 | 5,4 | 4,15 | 3,7 | 2,0 | 4,1 | |
| 7 | | | | Br | 151 | 6,45 | 10,6 | 7,8 | 5,4 | 4,15 | 3,7 | 2,0 | 4,0 | |
| 8 | | | | J | 145 | 6,4 | 10,55 | 7,8 | 5,4 | 4,15 | 3,75 | 2,0 | 4,1 | |
| 9 | | | | $N_3$ | 198 | 6,45 | 10,6 | 7,8 | 5,4 | 4,15 | 3,7 | 2,0 | 4,0 | |
| 10 | | | | $NH_2$ | 144 | 6,45 | 10,6 | 7,8 | 5,4 | 4,15 | 3,1 | 2,0 | 3,45 | |
| | | | | | | | | | s, 2H $NCH_2O$ | m, 1H $OCH(CH_2)_2$ | m, 2H $CH_2OAc$ | | | |
| 14 | OH | H | O | $OCH(CH_3)_2$ | 196 | 6,45 | 10,6 | 7,8 | 5,3 | 4,15 | 3,5 | – | 4,0 | $OCH(CH_3)_2$ 3,35/1,02 |
| 16 | | | | $OCH(CH_3)C_2H_5$ | 200 | 6,45 | 10,6 | 7,8 | 5,3 | 4,15 | 3,5 | – | 4,0 | $OCH(CH_3)C_2H_5$ 3,4/1,0 |

Tabelle 2, Forts.

| Formel I: No. | $R^1$ | $R^2$ | X | $R^3$ | Fp. °C | NMR δ-Werte in Dimethylsulfoxyd ($Me_2SO-d_6$, 270 MHz): s,2H $NH_2$ C-2 | s,1H C-6 | s,1H CH C-8 | s,2H $NCH_2O$ | m,1H $OCH(CH_2)_2$ | m,2H $CH_2OAc$ | s,3H $OCOCH_3$ | m,2H $CH_2R^3$ | m $R^3$ | |
|---|---|---|---|---|---|---|---|---|---|---|---|---|---|---|---|
| 17 | OH | Ac | O | $OCH_3$ | 214 | 6,4 | 10,55 | 7,8 | 5,3 | 4,15 | 3,6 | 1,9 | 4,0 | $OCH_3$ | 3,15 |
| 18 | | | | $OC_2H_5$ | 220 | 6,45 | 10,6 | 7,75 | 5,3 | 4,15 | 3,6 | 1,9 | 4,0 | $OC_2H_5$ | 3,3/1,0 |
| 19 | | | | $O(CH_2)_2CH_3$ | 216 | 6,45 | 10,6 | 7,8 | 5,3 | 4,15 | 3,6 | 1,9 | 4,0 | $O(CH_2)_2CH_3$ | 1,4/0,8 |
| 20 | | | | $OCH(CH_3)_2$ | 232 | 6,45 | 10,6 | 7,7 | 5,35 | 4,15 | 3,55 | 1,9 | 3,9 | $OCH(CH_3)_2$ | 3,35/1,[illegible] |
| 21 | | | | $O(CH_2)_3CH_3$ | 213 | 6,45 | 10,6 | 7,8 | 5,3 | 4,15 | 3,6 | 1,9 | 3,9 | $O(CH_2)_3CH_3$ | 1,5/0,8 |
| 22 | | | | $OCH(CH_3)C_2H_5$ | 219 | 6,45 | 10,55 | 7,8 | 5,3 | 4,15 | 3,55 | 1,9 | 3,9 | $OCH(CH_3)C_2H_5$ | 3,4/1,[illegible] |
| 23 | | | | $OCH_2CH(CH_3)_2$ | 217 | 6,45 | 10,6 | 7,8 | 5,35 | 4,15 | 3,6 | 2,0 | 4,0 | $CH_3$<br>$OCH_2CH$<br>$CH_3$ | 3,1<br>3,4<br>0,8 |
| 24 | | | | $OC(CH_3)_3$ | 185 | 6,45 | 10,6 | 7,8 | 5,3 | 4,15 | 3,6 | 1,9 | 4,0 | $OC(CH_3)_3$ | 1,1 |
| 25 | | | | $O(CH_2)_4CH_3$ | 210 | 6,45 | 10,6 | 7,8 | 5,3 | 4,15 | 3,6 | 1,9 | 4,0 | $O(CH_2)_4CH_3$ | 1,7/0,8 |
| 26 | | | | $OCH(C_2H_5)_2$ | 221 | 6,45 | 10,6 | 7,8 | 5,35 | 4,15 | 3,45 | 1,85 | 4,0 | $C_2H_5$<br>$OCH$<br>$C_2H_5$ | 3,4<br>1,2<br>0,8 |
| 27 | | | | 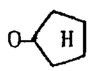 | 212 | 6,5 | 10,6 | 7,8 | 5,4 | 4,15 | 3,6 | 1,9 | 4,0 | 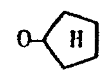 | 1,5 |

Tabelle 2, Forts.

| No. | Formel I $R^1$ | $R^2$ | X | $R^3$ | Fp. °C | s,2H $NH_2$ C-2 | s,1H C-6 | s,1H CH C-8 | s,2H $NCH_2O$ | m,1H $OCH(CH_2)_2$ | m,2H $CH_2OAc$ | s,3H $OCOCH_3$ | m,2H $CH_2R^3$ | m $R^3$ | |
|---|---|---|---|---|---|---|---|---|---|---|---|---|---|---|---|
| | | | | | | NMR δ-Werte in Dimethylsulfoxyd ($Me_2SO$-$d_6$, 270 MHz) | | | | | | | | | |
| 28 | OH | Ac | O | $OCH_2CH=CH_2$ | 209 | 6,4 | 10,6 | 7,8 | 5,3 | 4,15 | 3,4 | 1,95 | 3,9 | $OCH_2CH=CH_2$ | 5,0-6,0 |
| 29 | | | | $OCH_2C_6H_5$ | 186 | 6,5 | 10,65 | 7,8 | 5,35 | 4,15 | 3,4 | 1,95 | 4,0 | $OCH_2-C_6H_5$<br>$-C_6H_5$ | 4,45<br>7,3 |
| 30 | | | | $OCH(CH_3)C_6H_5$ | 180 | 6,45 | 10,6 | 7,7 | 5,35 | 4,15 | 3,55 | 1,85 | 4,0 | $CH_3$<br>$OCH$<br>$C_6H_5$ | 1,25<br>4,4<br>7,3 |
| 31 | | | | $OCH_2CF_3$ | 208 | 6,45 | 10,6 | 7,8 | 5,3 | 4,15 | 3,6 | 1,95 | 3,9 | $OCH_2CF_3$ | 3,2 |

Tabelle 2, Forts.

| No. | $R^1$ | $R^2$ (Formel I) | X | $R^3$ | Fp. °C | s,2H $NH_2$ C-2 | s,1H C-6 | s,1H CH C-8 | s,2H $NCH_2X$ | m,1H $XCH(CH_2)_2$ | m,2H $CH_2OR^2$ | $OR^2$ | m,2H $CH_2R^3$ | m,1H $CH(CH_3)_2$ | d,6H $CH(CH_3)$ |
|---|---|---|---|---|---|---|---|---|---|---|---|---|---|---|---|
| 32 | OH | H | O | $SCH(CH_3)_2$ | 187 | 6,45 | 10,6 | 7,8 | 5,35 | 4,15 | 2,8 | - | 3,5 | 3,2 | 1,1 |
| 33 | | | | $SOCH(CH_3)_2$ | 118 | 6,45 | 10,6 | 7,8 | 5,4 | 4,15 | 2,8 | - | 3,7 | 3,4 | 1,1 |
| 34 | | | | $SO_2CH(CH_3)_2$ | 146 | 6,45 | 10,6 | 7,8 | 5,4 | 4,15 | 2,8 | - | 3,8 | 3,5 | 1,1 |
| 35 | OH | Ac | O | $SCH(CH_3)_2$ | 198 | 6,45 | 10,6 | 7,8 | 5,4 | 4,15 | 3,6 | 1,9 | 4,0 | 3,2 | 1,2 |
| 36 | | | | $SOCH(CH_3)_2$ | 127 | 6,45 | 10,6 | 7,8 | 5,4 | 4,3 | 3,7 | 1,9 | 4,2 | 3,4 | 1,2 |
| 37 | | | | $SO_2CH(CH_3)_2$ | 157 | 6,45 | 10,6 | 7,8 | 5,4 | 4,3 | 3,7 | 1,9 | 4,3 | 3,5 | 1,2 |
| 38 | OH | H | S | $OCH(CH_3)_2$ | 196 | 6,4 | 10,5 | 7,7 | 5,1 | 4,15 | 2,7 | - | 3,5 | 3,35 | 1,1 |
| 39 | OH | Ac | S | $OCH(CH_3)_2$ | 205 | 6,4 | 10,5 | 7,7 | 5,1 | 4,15 | 3,6 | 1,9 | 3,35 | 3,35 | 1,1 |
| | | | | | | | s,1-2H C-6 | | s,2H $NCH_2O$ | m,1H $OCH(CH_2)_2$ | | s,3H $OCOCH_3$ | m,2H $CH_2OC_3H_7$ | | |
| 40 | H | Ac | O | $OCH(CH_3)_2$ | 89 | 5,53 | 8,72 | 7,88 | 5,13 | 4,3 | 3,5 | 2,05 | 3,72 | 3,4 | 1,08 |
| 41 | Cl | Ac | O | " | 122 | 4,8 | - | 7,9 | 5,45 | 4,3 | 3,5 | 2,1 | 3,7 | 3,4 | 1,05 |
| 42 | $NH_2$ | Ac | O | " | 168 | 6,68 | 7,75 | 8,02 | 5,38 | 4,15 | 3,6 | 1,98 | 3,9 | 3,4 | 1,0 |
| 43 | SH | Ac | O | " | 192 | 6,9 | 13,1 | 8,2 | 5,35 | 4,15 | 3,5 | 1,85 | 3,9 | 3,4 | 1,0 |

NMR δ-Werte in Dimethylsulfoxyd ($Me_2SO-d_6$, 270 MHz)

Tabelle 2, Forts.

| No. | Formel I $R^1$ | $R^2$ | X | $R^3$ | Fp. °C | NMR δ-Werte in Dimethylsulfoxyd ($Me_2SO-d_6$, 270 MHz) s,2H $NH_2$ C-2 | s,1H C-6 | s,1H CH C-8 | s,2H $NCH_2O$ | m,1H $OCH(CH_2)_2$ | m,2H $CH_2OAc$ | s,3H $OCOCH_3$ | m,2H $CH_2R^3$ | m,1H $OCH(CH_3)_2$ | d,6H $OCH(CH_3)_2$ |
|---|---|---|---|---|---|---|---|---|---|---|---|---|---|---|---|
| 44 | $OCH(CH_3)_2$ | Ac | O | $OCH(CH_3)_2$ | 137 | 6,4 | s,1H $OCH(CH_3)_2$ 5,3<br>d,6H $OCH(CH_3)_2$ 1,35 | 8,0 | 5,5 | 4,15 | 3,4 | 1,8 | 4,0 | 3,6 | 1,0 |
| | | | | | | | | | | | | | | m $R^3$ | |
| 45 | $OCH_3$ | Ac | O | $OCH(CH_3)_2$ | 96 | 6,4 | - | 7,9 | 5,4 | 4,0 | 3,4 | 1,9 | 3,9 | $OCH(CH_3)_2$ | 3,35/1,0 |
| 46 | 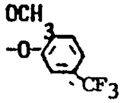 | Ac | O | $OCH(CH_3)_2$ | Öl | 5,0 | 7,4 | 7,8 | 5,5 | 4,1 | 3,7 | 2,0 | 3,7 | $OCH(CH_3)_2$ | 3,35/1,1 |
| 47 | $N_3$ | Ac | O | $OCH(CH_3)_2$ | 116 | 8,4 | - | 8,3 | 5,6 | 4,1 | 3,4 | 1,9 | 3,7 | $OCH(CH_3)_2$ | 3,35/0,9 |
| 48 | H | $-CH(CH_3)_2$ | O | $OCH(CH_3)_2$ | 81 | 6,5 | 8,7 | 8,2 | 5,6 | 3,8 | 3,3 | - | 3,5 | $2OCH(CH_3)_2$ | 3,35/1,0 |
| | | | | | | | | | | | | | | m2(1H) $OCH(CH_2)_2$ | d2(6H) $OCH(CH_2)$ |
| 49 | OH | $-CH(CH_3)_2$ | O | $OCH(CH_3)_2$ | 222 | 6,4 | 10,55 | 7,75 | 5,35 | 4,15 | | | | 3,6 | 1,0 |

Tabelle 2, Forts.

| No. | Formel I $R^1$ | $R^2$ | X | $R^3$ | Fp. °C | NMR δ-Werte in Dimethylsulfoxyd ($Me_2SO-d_6$, 270 MHz) s,2H $NH_2$ C-2 | s,1H C-6 | s,1H CH C-8 | s,2H $NCH_2O$ | m,1H $OCH(CH_2)_2$ | m,2H $CH_2OAc$ | s,3H $OCOCH_3$ | m,2H $CH_2R^3$ | m,1H $OCH(CH_3)_2$ | d,6H $OCH(CH_3)_2$ |
|---|---|---|---|---|---|---|---|---|---|---|---|---|---|---|---|
| | | | | | | | | | | | m,4H $CH_2OR^{2+3}$ | | m $R^3$ | | |
| 50 | OH | $-CH_2-CH=CH_2$ | O | $-OCH_2CH=CH_2$ | 216 | 6,45 | 10,65 | 7,8 | 5,4 | 4,1 | 3,9/3,4 | | $2OCH_2CH=CH_2$ | | 5,0-6,0 |
| 51 | OH | $-CH_2CF_3$ | O | $-OCH_2CF_3$ | 212 | 6,4 | 10,6 | 7,8 | 5,4 | 4,3 | 3,7 | | m,4H<br>$2OCH_2CF_3$ | | 4,0 |
| 52 | $-OCH(CH_3)_2$ | $-CH(CH_3)_2$ | O | $-OCH(CH_3)_2$ | ~100 | 6,35 | - | 7,95 | 5,6 | 4,1 | 3,8/3,4 | | m $R^3$<br>$3OCH(CH_3)_2$ | | 3,35/1,0 |

## Ansprüche

1. Verbindungen der Formel 1

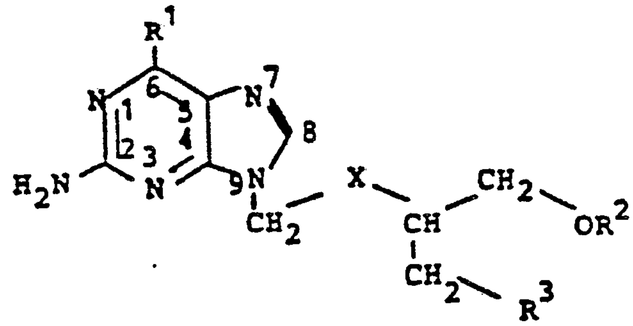

worin

$R^1$ H, Halogen, OH, SH, Azido, $NH_2$ oder Z-$R^5$ ist, wobei bedeuten Z O, S, SO, $SO_2$ oder NH und $R^5$ einen acyclischen aliphatischen Kohlenwasserstoffrest mit 1 bis 6 C-Atomen, welcher a) unsubstituiert ist oder b) durch wenigstens eines der Merkmale modifiziert ist, daß er eine Alkoxygruppe mit 1 bis 3 C-Atomen oder eine Doppelbindung enthält oder mindestens teilweise fluoriert ist, oder Cycloalkyl mit 3 bis 6 C-Atomen oder Phenylalkyl, das direkt an Z oder über eine Alkylengruppe von 1 bis 3 C-Atomen gebunden ist oder Phenyl, wobei der Phenylring jeweils höchstens zweifach, vorzugsweise höchstens einmal durch einen Substituenten aus der Gruppe Halogen, Trifluormethyl,Alkoxy und: Alkylthio mit jeweils 1 bis 3 C-Atomen substituiert kann,

$R^2$ H ist oder die Bedeutung von $R^5$ hat oder eine Acylgruppe CO-$R^6$ ist, wobei $R^6$ Alkyl mit 1 bis 6 C-Atomen, Benzyl oder Phenyl ist,`

$R^3$ Halogen, Azido, unsubstituiertes Amino oder den Rest Y-$R^4$ bedeutet, wobei $R^4$ dieselbe Bedeutung wie $R^5$ hat und Y ebenso wie

X O, S, SO oder $SO_2$ bedeutet und X und Y gleich oder verschieden sein können, wobei 9-(1,3-Bis-(isopropoxy)-propyl-2-oxymethyl-guanin und solche Verbindungen ausgenommen sind, in denen nebeneinander $R^1$ OH oder SH, $R^2$ H, $R^3$ Y-$R^4$, X O oder S, Y O und $R^4$ Alkyl mit 1 bis 6 C-Atomen sind bzw. in denen nebeneinander $R^1$ OH oder $NH_2$, Benzyl, $R^3$ Y-$R^4$ mit Y=O und $R^4$ = Benzyl und X O ist.

2. Verbindungen nach Anspruch 1, dadurch gekennzeichnet, daß

$R^1$ H, Halogen, OH, SH oder $NH_2$

$R^2$ H oder Acyl,

$R^3$ Halogen oder Y-$R^4$

X und Y O, S, SO oder $SO_2$ und gleich oder verschieden und

$R^4$ einen acyclischen aliphatischen Kohlenwasserstoffrest mit 1 bis 6 C-Atomen, wenigstens teilweise fluoriertes. Alkyl mit 1-4 C-Atomen oder Cycloalkyl mit 3-4 C-Atomen darstellen,

jedoch mit Ausnahme solcher Verbindungen, in denen nebeneinander $R^1$ OH oder SH, $R^2$ H, $R^3$ y-$R^4$, X O oder S, Y O und $R^4$ Alkyl mit 1-6 C-Atomen sind.

3. Verbindungen nach Anspruch 1 oder 2, dadurch gekennzeichnet, daß $R^4$ teilweise fluoriertes Alkyl mit 1-4 C-Atomen oder Cycloalkyl mit 3-4 C-Atomen ist.

4. Verbindungen nach Anspruch 1 oder 2, dadurch gekennzeichnet, daß Y S, SO oder $SO_2$ ist.

5. Verbindungen nach einem oder mehreren der Ansprüche 1 bis 4, dadurch gekennzeichnet, daß $R^1$ H, Halogen, SH oder $NH_2$ ist.

6. Verbindungen nach einem oder mehreren der Ansprüche 1 bis 4, dadurch gekennzeichnet, daß $R^1$ H, OH oder $NH_2$, $R^2$ Acyl, insbesondere Acetyl,und X O oder S sind.

7. Verbindungen nach einem oder mehreren der Ansprüche 1 bis 5, dadurch gekennzeichnet, daß in den Resten $R^4$ bis $R^6$ der acyclische Kohlenwasserstoffrest 3 bis 5 C-Atome und der cycloaliphatische Rest 4 bis 6 und insbesondere 5 bis 6 C-Atome, in $R^1$ etwa enthaltenes Halogen Chlor oder Brom und in $R^3$

etwa enthaltenes Halogen Fluor oder Chlor ist.

8. Arzneimittel, gekennzeichnet durch einen Gehalt an einer wirksamen Menge wenigstens einer Verbindung gemäß einem oder mehreren der Ansprüche 1 bis 7.

9. Verfahren zur Herstellung von Verbindungen gemäß einem oder mehreren der Ansprüche 1 bis 7, dadurch gekennnzeichnet, daß man unter üblichen Bedingungen
A) zur Herstellung von Verbindungen, in denen $R^1$ Hydroxy, Amino- oder Wasserstoff ist, 2,6,9-Tris-(trialkylsilyl)- bzw. 2,9-Bis-(trialkylsilyl)-2-amino-purine gemäß einer der Formeln 8, 11 und 12

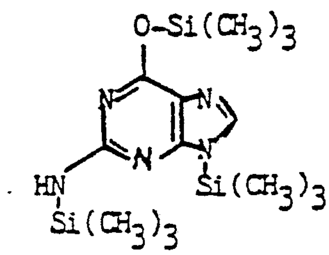

8

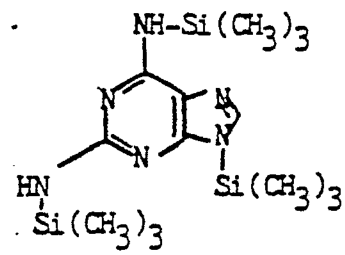

11

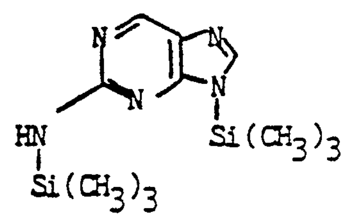

12

mit Halogen-, insbesondere Chlorverbindungen der Formel

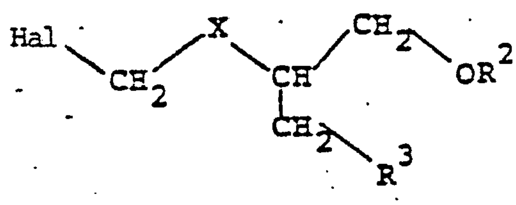

13

umsetzt, worin $R^2$ = Acyl ist und X und $R^3$ die in den Ansprüchen 1 bis 7 angegebene Bedeutung haben,und die Trialkylsilylschutzgruppen abspaltet, oder
B) zur Herstellung von Verbindungen, in denen $R^1$ Hydroxy, Mercapto, Amino oder Wasserstoff ist, entsprechende D1- bzw. Triacyl-Verbindungen der Formeln 9, 10, 6a bzw. 7a

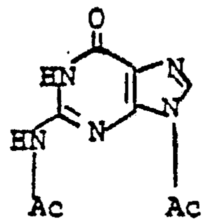

9

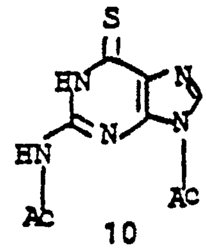

10

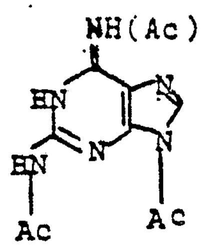

6a

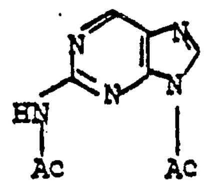

7a

mit reaktiven Acetoxy-Verbindungen der Formel 14

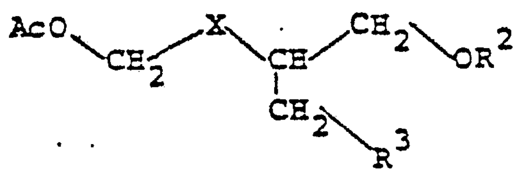

14

umsetzt, worin $R^2$ Acyl ist und X und $R^3$ die in den Ansprüchen 1 bis 7 angegebene Bedeutung haben, oder

C) zur Herstellung von Verbindungen, in denen $R^1$ Halogen ist, Verbindungen der Formel 5

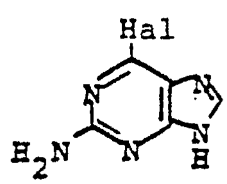

5

mit aktiven Halogen-Verbindungen der unter A angegbenen Formel 13 umsetzt und die so erhaltene Verbindung 18 isoliert oder durch saure Verseifung zu Verbindungen, in denen $R^1$ Hydroxy ist, oder durch Umsetzung mit Schwefelwasserstoff zu Verbindungen, In denen $R^1$ Mercapto ist, oder durch Umsetzung mit Alkaliaziden zu Verbindungen, in denen $R^1$ Azido ist, oder durch Umsetzung mit Ammoniak zu Verbundungen, in denen $R^1$ Amino ist, oder durch katalytische dehalogenierende Hydrierung zu Verbindungen, in denen $R^1$ Wasserstoff ist, oder durch Umsetzung mit Alkoholen bzw. Phenolen, Mercaptanen bzw. Thiophenolen oder Aminen bzw. Anilinen zu Verbindungen, in denen $R^1$ $ZR^5$ ist, umsetzt, oder

D) zur Herstellung von Verbindungen, in denen $R^2$ Wasserstoff ist, eine Verbindung, die noch einem der Verfahren A bis C hergestellt ist, verseift.

**10.** Verwendung von Verbindungen gemäß einem oder mehreren der Ansprüche 1 bis 7 oder von 9-(1,3-Bis-(isopropoxy)propyl-2-oxymethyl)-guanin zur Herstellung von Arzneimitteln zur Behandlung von durch Viren verursachten Krankheiten.

**11.** Verbindung nach Anspruch 1, dadurch gekennzeichnet, daß sie das 9-(1-Isopropoxymethyl-2-acetoxyäthoxymethyl)-guanin, das 2 Amino-9-(1-isopropoxymethyl-2-acetoxyäthoxymethyl)-purin, das 2-Amino-6-isopropoxy-9-(1-isopropoxymethyl-2-acetoxyäthoxymethyl)-purin, das 2-Amino-9-[1,3-bis-(isopropoxy)-propyl-2-oxymethyl]-purin oder das 2-Amino-6-isopropoxy-9-[1,3-bis-(isopropoxy)-propyl-2-oxymethyl]-purin ist.

**12.** 9-(1-Isopropoxymethyl-2-hydroxyäthoxymethyl)-guanin.

Patentanspruch für folgenden Vertragsstaat: AT

**1.** Verfahren zur Herstellung von Verbindungen der Formel 1

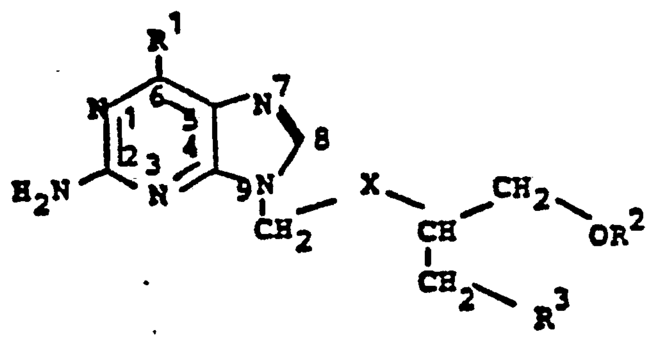

worin

$R^1$ H, Halogen, OH, SH, Azido, $NH_2$ oder Z-$R^5$ ist, wobei bedeuten Z O, S, SO, $SO_2$ oder NH und $R^5$ einen acyclishen aliphatischen Kohlenwasserstoffrest mit 1 bis 6 C-Atomen, welcher a) unsubstituiert ist oder b) durch wenigstens eines der Merkmale modifiziert, ist daß er eine Alkoxygruppe mit 1 bis 3 C-Atomen oder eine Doppelbindung enthält oder mindestens teilweise fluoriert ist, oder Cycloalkyl mit 3 bis 6 C-Atomen oder Phenylalkyl, das direkt an Z oder über eine Alkylengruppe von 1 bis 3 C-Atomen gebunden ist, oder phenyl, wobei der Phenylring jeweils höchstens zweifach, vorzugsweise höchstens einmal durch einen Substituenten aus der Gruppe Halogen, Trifluormethyl, Alkoxy und Alkylthio mit jeweils 1 bis 3 C-Atomen substituiert sein kann, $R^2$H ist oder die Bedeutung von $R^5$ hat oder eine Acylgruppe $COR^6$ ist, wobei $R^6$ Alkyl mit 1-6 C-Atomen, Benzyl oder Phenyl ist,

$R^3$ Halogen, Azido, unsubstituiertes Amino oder den Rest Y-$R^4$ bedeutet, wobei $R^4$ dieselbe Bedeutung wie $R^5$ hat und Y ebenso wie

X O, S, SO oder $SO_2$ bedeutet und X und Y gleich oder verschieden sein können, wobei 9-(1,3-Bis-isopropoxy)-propyl-2-oxymethyl)-guanin und solche Verbindungen ausgenommen sind, in denen nebeneinander $R^1$ OH oder SH, $R^2$ H, $R^3$ Y-$R^4$, Y O oder S, Y O und $R^4$ Alkyl mit 1 bis 6 C-Atomen sind bzw. in denen nebeneinander $R^1$ OH oder $NH_2$, $R^2$ Benzyl Y-$R^4$ mit Y = O und $R^4$ Benzyl und X O ist, dadurch gekennzeichnet, daß man unter üblichen Bedingungen

A) zur Herstellung von Verbindungen, in denen $R^1$ Hydroxy, Amino- oder Wasserstoff ist, 2,6,9-Tris-(trialkylsilyl)- bzw. 2,9-Bis-(trialkylsilyl)-2-amino-purine gemäß einer der Formeln 8, 11 und 12

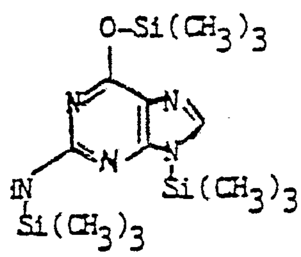

8

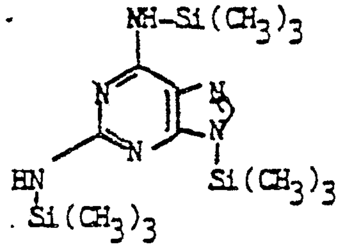

11

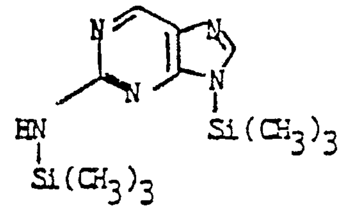

12

mit Halogen-, insbesondere Chlorverbindungen der Formel

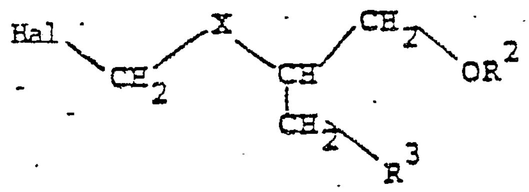

13

umsetzt, worin $R^2$ = Acyl ist und X und $R^3$ die angegebene Bedeutung haben, und die Trialkylsilylschutzgruppen abspaltet oder

B) zur Herstellung von Verbindungen, in denen $R^1$ Hydroxy, Mercapto, Amino oder Wasserstoff ist, entsprechende D1- bzw. Triacyl-Verbindungen der Formeln 9, 10, 6a bzw. 7a

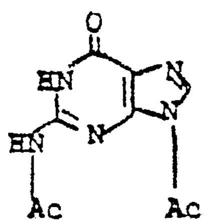

9

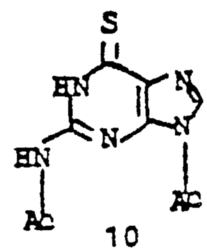

10

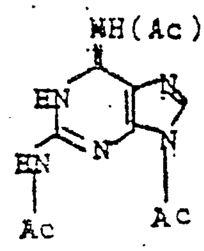

6a

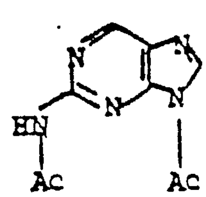

7a

mit reaktiven Acetoxy-Verbindungen der Formel 14

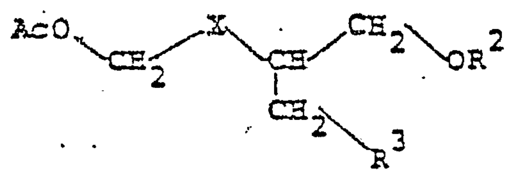

14

umsetzt, worin $R^2$ Acyl ist und X und $R^3$ die oben angegebene Bedeutung haben, oder
C) zur Herstellung von Verbindungen, in denen $R^1$ Halogen ist, Verbindungen der Formel 5

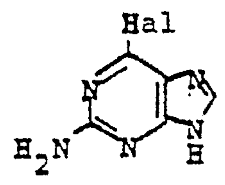

5

mit aktiven Halogen-Verbindungen der unter A angegebenen Formel 13 umsetzt und die so erhaltene Verbindung 18 isoliert oder durch saure Verseifung zu Verbindungen, in denen $R^1$ Hydroxy ist, oder durch Umsetzung mit Schwefelwasserstoff zu Verbindungen, In denen $R^1$ Mercapto ist, oder durch Umsetzung mit Alkaliaziden zu Verbindungen, in denen $R^1$ Azido ist, oder durch Umsetzung mit Ammoniak zu Verbindungen, in denen $R^1$ Amino ist, oder durch Katalytische dehalogenierende Hydrierung zu Verbindungen, in denen $R^1$ Wasserstoff ist, oder durch Umsetzung mit Alkoholen bzw. phenolen, Mercaptanen bzw. Thiophenolen oder Aminen bzw. Anilinen zu Verbindungen, in denen $R^1$ $ZR^5$ ist, umsetzt oder
D) zur Herstellung von Verbindungen, in denen $R^2$ Wasserstoff ist, eine Verbindung, die nach einem der Verfahren A bis C bergestellt ist, verseift.

**2.** Verfahren nach Anspruch 1, dadurch gekennzeichnet, daß Verbindungen hergestellt werden, in denen

| | |
|---|---|
| $R^1$ | H, Halogen, OH, SH oder $NH_2$ |
| $R^2$ | H oder Acyl, |
| $R^3$ | Halogen oder Y-$R^4$ |
| X und Y | O, S, SO oder $SO_2$ und gleich oder verschieden und |
| $R^4$ | einen acyclischen aliphatischen Kohlenwasserstoffrest mit 1 bis 6 C-Atomen, wenigstens teilweise fluoriertes Alkyl mit 1-4 C-Atomen oder Cycloalkyl mit 3-4 C-Atomen darstellen, |

jedoch mit Ausnahme Solcher Verbindungen, in denen nebeneinander $R^1$ OH oder SH, $R^2$ H, $R^3$ Y-$R^4$, X O oder S, Y O und $R^4$ Alkyl mit 1-4 C-Atomen sind.

**3.** Verfahren nach Anspruch 1 oder 2, dadurch gekennzeichnet, daß Verbindungen hergestellt werden, in denen $R^4$ teilweise fluoriertes Alkyl mit 1-4 C-Atomen oder Cycloalkyl mit 3-4 C-Atomen ist.

**4.** verfahren nach Anspruch 1 oder 2, dadurch gekennzeichnet, daß Verbindungen hergestellt werden, in denen Y S, SO oder $SO_2$ ist.

**5.** Verfahren nach einem oder mehreren der Ansprüche 1 bis 4, dadurch gekennzeichnet, daß Verbindungen hergestellt werden, in denen $R^1$ H, Halogen, SH oder $NH_2$ ist.

**6.** Verfahren nach einem oder mehreren der Ansprüche 1 bis 4, dadurch gekennzeichnet, daß Verbindungen hergestellt werden, in denen $R^1$ H, OH oder $NH_2$, $R^2$ Acyl, insbesondere Acetyl, und X O oder S sind.

**7.** Verfahren nach einem odermehreren der Ansprüche 1 bis 5, dadurch gekennzeichnet, daß Verbindungen hergestellt werden, in denen in $R^4$ bis R6 der acyclische Kohlenwasserstoffrest 3 bis 5 C-Atome und der cycloaliphatische Rest 4 bis 6 und insbesondere 5 bis 5 C-Atome, in $R^1$ etwa enthaltenes Halogen Chlor oder Brom und in $R^3$ etwa enthaltenes Halogen Fluor oder Chlor ist.

8. Verfahren zur Herstellung einer pharmazeutischen Komposition, die zur Behandlung von durch Viren verursachten Krankheiten geeignet ist, dadurch gekennzeichnet, daß man als aktive Substanz eine Verbindung der Formel 1 einarbeitet, die nach dem Verfahren gemäß einem oder mehreren der Ansprüche 1 bis 7 hergestellt ist, in Kombination mit einem pharmazeutisch unbedenklichen Träger derart, daß man eine für die Verabreichung geeignete Komposition erhält.

9. Verfahren zur Herstellung von Arzneimittelformulierungen zur Behandlung von durch Viren verursachten Krankheiten, dadurch gekennzeichnet, daß man eine Verbindung 1, die nach dem Verfahren gemäß einem oder mehreren der Ansprüche 1 bis 7 erhalten worden ist, mit den üblichen Träger- und Hilfsstoffen zu Arzneimitteln formuliert.

10. Verfahren nach Anspruch 1, dadurch gekennzeichnet, das folgende Verbindungen hergestellt werden:
9-(1-Isopropoxymethyl-2-acetoxyäthoxymethyl)-guanin,
2-Amino-9-(1-isopropoxymethyl-2-acetoxyäthoxymethyl)-purin,
2-Amino-6-isopropoxy-9-(1-isopropoxymethyl-2-acetoxyäthoxymethyl)-purin, 2-Amino-9-[1,3-bis-(isopropoxy)-propyl-2-oxymethyl]-purin und 2-Amino-6-isopropoxy-9-[1,3-bis-(isopropoxy)-propyl-2-oxymethyl]-purin.

11. Verfahren nach Anspruch 1, dadurch gekennzeichnet, daß 9-(1-Isopropoxymethyl-2-hydroxyäthoxymethyl)-guanin hergestellt wird.

## Claims

1. A compound of the formula 1

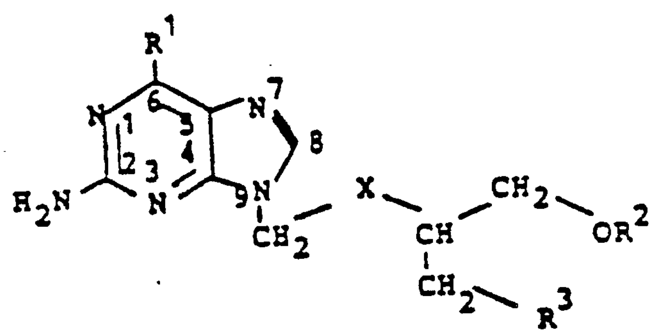

in which

$R^1$ is H, halogen, OH, SH, azido, $NH_2$ or Z-$R^5$, Z denoting O, S, SO, $SO_2$ or NH, and $R^5$ denoting an acyclic aliphatic hydrocarbon radical which has 1 to 6 carbon atoms and which a) is unsubstituted or b) is modified by at least one of the features that it contains an alkoxy group having 1 to 3 carbon atoms or a double bond, or it is at least partially fluorinated, or cycloalkyl having 3 to 6 carbon atoms, or phenylalkyl which is bonded to Z either directly or via an alkylene group of 1 to 3 carbon atoms, or phenyl, it being possible for each phenyl ring to be substituted not more than twice, preferably not more than once, by a substituent from the group comprising halogen, trifluoromethyl, alkoxy or alkylthio having, in each case, 1 to 3 carbon atoms,

$R^2$ is H or has the meaning of $R^5$ or is an acyl group CO-$R^6$, $R^6$ being alkyl having 1 to 6 carbon atoms, benzyl or phenyl,

$R^3$ denotes halogen, azido, unsubstituted amino or the radical Y-$R^4$, $R^4$ having the same meaning as $R^5$, and Y denoting, in the same way as

X denotes O, S, SO or $SO_2$, and X and Y can be identical or different, an exception being made of 9-(1,3-bis-(isopropoxy)-propyl-2-oxymethyl)-guanine and those compounds in which side by side $R^1$ is OH or SH, $R^2$ is H, $R^3$ is Y-$R^4$, X is O or S, Y is O, and $R^4$ is alkyl having 1 to 6 carbon atoms, or in which side by side $R^1$ is OH or $NH_2$, $R^2$ is benzyl, $R^3$ is Y-$R^4$ with Y = O and $R^4$ = benzyl and X is O.

2. A compound as claimed in claim 1, wherein

$R^1$ represents H, halogen, OH, SH or $NH_2$,
$R^2$ represents H or acyl,
$R^3$ represents halogen or Y-$R^4$,
X and Y represent O, S, SO or $SO_2$ and are identical or different, and
$R^4$ represents an acyclic aliphatic hydrocarbon radical having 1 to 6 carbon atoms, at least partially fluorinated alkyl having 1-4 carbon atoms, or cycloalkyl having 3-4 carbon atoms,
but with the exception of those compounds in which side by side $R^1$ is OH or SH, $R^2$ is H, $R^3$ is Y-$R^4$, X is O or S, Y is O and $R^4$ is alkyl having 1-6 carbon atoms.

**3.** A compound as claimed in claim 1 or 2, wherein $R^4$ is partially fluorinated alkyl having 1-4 carbon atoms, or cycloalkyl having 3-4 carbon atoms.

**4.** A compound as claimed in claim 1 or 2, wherein Y is S, SO or $SO_2$.

**5.** A compound as claimed in one or more of claims 1 to 4, wherein $R^1$ is H, halogen, SH or $NH_2$.

**6.** A compound as claimed in one or more of claims 1 to 4, wherein $R^1$ is H, OH or $NH_2$, $R^2$ is acyl, in particular acetyl, and X is O or S.

**7.** A compound as claimed in one or more of claims 1 to 5, wherein, in the radicals $R^4$ to $R^6$, the acyclic hydrocarbon radical has 3 to 5 carbon atoms and the cycloaliphatic radical has 4 to 6 and, in particular 5 to 6 carbon atoms, and any halogen contained in $R^1$ is chlorine or bromine, and any halogen contained in $R^3$ is fluorine or chlorine.

**8.** A medicament which contains an effective amount of at least one compound as claimed in one or more of claims 1 to 7.

**9.** A process for the preparation of compounds as claimed in one or more of claims 1 to 7, which comprises, under the usual conditions,
A) for the preparation of compounds in which $R^1$ is hydroxyl, amino or hydrogen, reacting 2,6,9-tris-(trialkylsilyl)-or 2,9-bis(trialkylsilyl)-2-aminopurines as represented by one of the formulae 8, 11 and 12.

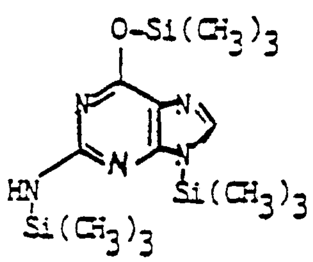
8

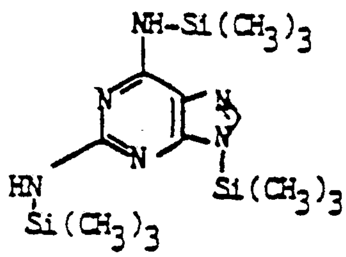
11

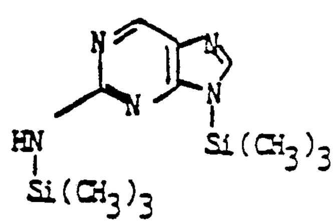
12

with halogen, in particular chlorine, compounds of the formula

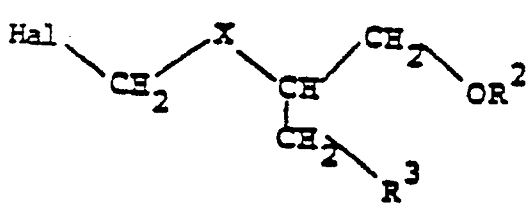
13

in which
$R^2$ is acyl, and
X and $R^3$ have the meaning indicated in claims 1 to 7, and eliminating the trialkylsilyl protective groups or
B) for the preparation of compounds in which $R^1$ is hydroxyl, mercapto, amino or hydrogen, reacting appropriate diacyl or triacyl compounds of the formulae 9, 10, 6a or 7a

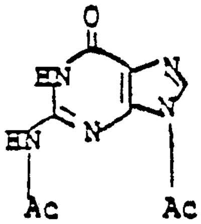
9

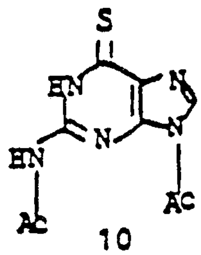
10

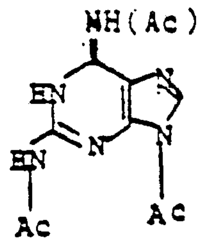
6a

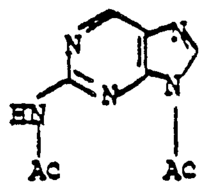
7a

with reactive acetoxy compounds of the formula 14

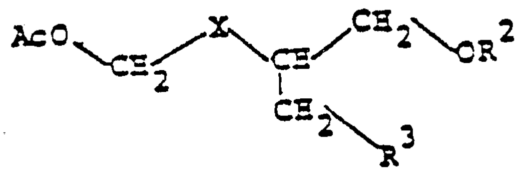
14

in which
$R^2$ is acyl, and
X and $R^3$ have the meaning indicated in claims 1 to 7, or
C) for the preparation of compounds in which $R^1$ is halogen, reacting compounds of the formula 5

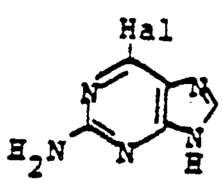
5

with active halogen compounds of the formula 13 indicated under A, and isolating the resulting compound 18, or converting them by acid hydrolysis into compounds in which $R^1$ is hydroxyl, or converting them by reaction with hydrogen sulfide into compounds in which $R^1$ is mercapto, or converting them by reaction with alkali metal azides into compounds in which $R^1$ is azido, or converting them by reaction with ammonia into compounds in which $R^1$ is amino, or converting them by catalytic dehalogenating hydrogenation into compounds in which $R^1$ is hydrogen, or converting

them by reaction with alcohols or phenols, mercaptans or thiophenols, or amines or anilines into compounds in which $R^1$ is $ZR^5$, or

D) for the preparation of compounds in which $R^2$ is hydrogen, hydrolyzing a compound which has been prepared by one of processes A to C.

**10.** The use of compounds as claimed in one or more of claims 1 to 7 or of 9-(1,3-bis-(isopropoxy)propyl-2-oxymethyl)-guanine for the preparation of medicaments for treating diseases caused by viruses.

**11.** A compound as claimed in claim 1, which is 9-(1-isopropoxymethyl-2-acetoxyethoxymethyl)-guanine, 2-amino-9-(1-isopropoxymethyl-2-acetoxyethoxymethyl)-purine, 2-amino-6-isopropoxy-9-(1-isopropoxymethyl-2-acetoxyethoxy-methyl)-purine, 2-amino-9-[1,3-bis-(isopropoxy)-propyl-2-oxymethyl]-purine or 2-amino-6-isopropoxy-9-[1,3-bis-(isopropoxy)-propyl-2-oxymethyl]-purine.

**12.** 9-(1-isopropoxymethyl-2-hydroxyethoxymethyl)-guanine.

Claim for the Contracting State: AT

**1.** A process for the preparation of a compound of the formula 1

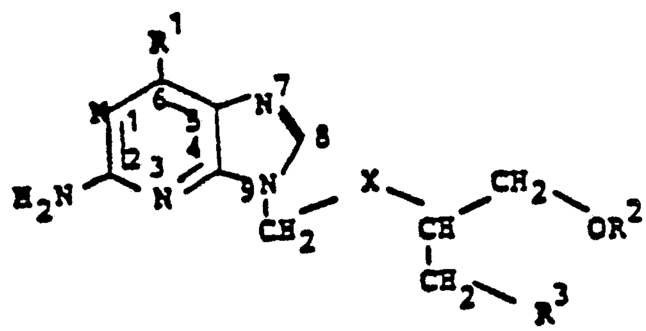

in which 5

- $R^1$ is H, halogen, OH, SH, azido, $NH_2$ or Z-$R^5$, Z denoting O, S, SO, $SO_2$ or NH, and $R^5$ denoting an acyclic aliphatic hydrocarbon radical which has 1 to 6 carbon atoms and which a) is unsubstituted or b) is modified by at least one of the features that it contains an alkoxy group having 1 to 3 carbon atoms or a double bond, or it is at least partially fluorinated, or cycloalkyl having 3 to 6 carbon atoms, or phenylalkyl which is bonded to Z either directly or via an alkylene group of 1 to 3 carbon atoms, or phenyl, it being possible for each phenyl ring to be substituted not more than twice, preferably not more than once, by a substituent from the group comprising halogen, trifluoromethyl, alkoxy or alkylthio having, in each case, 1 to 3 carbon atoms,
- $R^2$ is H or has the meaning of $R^5$ or is an acyl group CO-$R^6$, $R^6$ being alkyl having 1 to 6 carbon atoms, benzyl or phenyl,
- $R^3$ denotes halogen, azido, unsubstituted amino or the radical y-$R^4$, $R^4$ having the same meaning as $R^5$, and Y denoting, in the same way as
- X denotes O, S, SO or $SO_2$, and X and Y can be identical or different, an exception being made of 9-(1,3-bis-(isopropoxy)-propyl-2-oxymethyl)-guanine and those compounds in which side by side $R^1$ is OH or SH, $R^2$ is H, $R^3$ is Y-$R^4$, X is O or S, Y is O, and $R^4$ is alkyl having 1 to 6 carbon atoms, or in which side by side $R^1$ is OH or $NH_2$, $R^2$ is benzyl, $R^3$ is Y-$R^4$ with Y = O and $R^4$ = benzyl and X is O, which comprises, under the usual conditions,

A) for the preparation of compounds in which $R^1$ is hydroxyl, amino or hydrogen, reacting 2,6,9-tris-(trialkylsilyl)-or 2,9-bis(trialkylsilyl)-2-aminopurines as represented by one of the formulae 8, 11 and 12

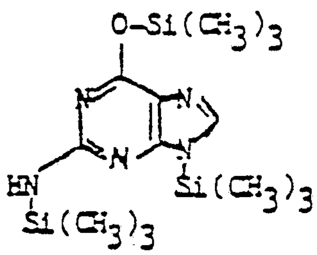

8

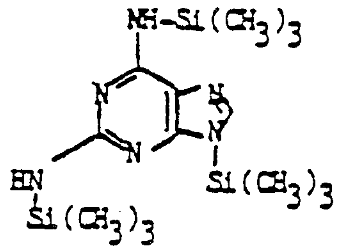

11

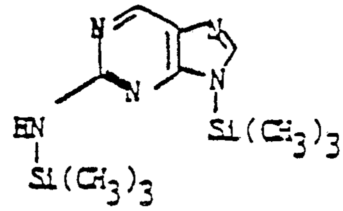

12

with halogen, in particular chlorine, compounds of the formula

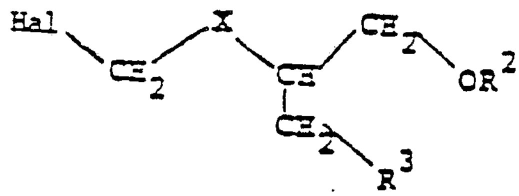

13

in which

$R^2$ is acyl, and X and $R^3$ have the meaning indicated and eliminating the trialkylsilyl protective groups or

B) for the preparation of compounds in which $R^1$ is hydroxyl, mercapto, amino or hydrogen, reacting appropriate diacyl or triacyl compounds of the formulae 9, 10, 6a or 7a

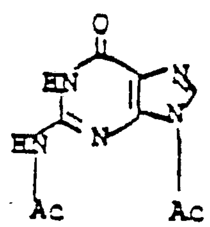

9

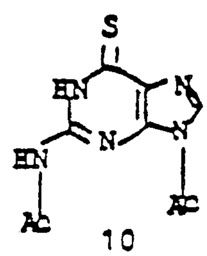

10

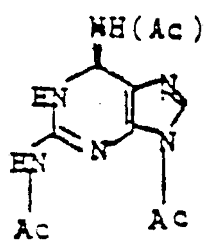

6a

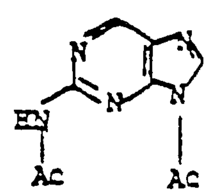

7a

with reactive acetoxy compounds of the formula 14

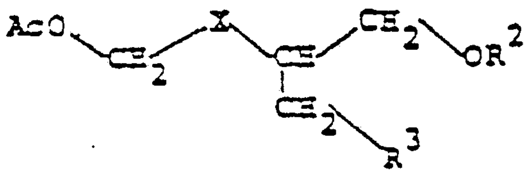

14

in which
$R^2$ is acyl, and X and $R^3$ have the meaning indicated above,
or
C) for the preparation of compounds in which $R^1$ is halogen, reacting compounds of the formula 5

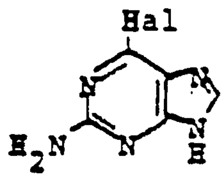

5

with active halogen compounds of the formula 13 indicated under A, and isolating the resulting compound 18, or converting them by acid hydrolysis into compounds in which $R^1$ is hydroxyl, or converting them by reaction with hydrogen sulfide into compounds in which $R^1$ is mercapto, or converting them by reaction with alkali metal azides into compounds in which $R^1$ is azido, or converting them by reaction with ammonia into compounds in which $R^1$ is amino, or converting them by catalytic dehalogenating hydrogenation into compounds in which $R^1$ is hydrogen, or converting them by reaction with alcohols or phenols, mercaptans or thiophenols, or amines or anilines into compounds in which $R^1$ is $ZR^5$, or
D) for the preparation of compounds in which $R^2$ is hydrogen, hydrolyzing a compound which has been prepared by one of processes A to C.

2. A process as claimed in claim 1, wherein compounds are prepared in which

| | |
|---|---|
| $R^1$ | represents H, halogen, OH, SH or $NH_2$, |
| $R^2$ | represents H or acyl, |
| $R^3$ | represents halogen or $Y\text{-}R^4$, |
| X and Y | represent O, S, SO or $SO_2$ and are identical or different, and |
| $R^4$ | represents an acyclic aliphatic hydrocarbon radical having 1 to 6 carbon atoms, at least partially fluorinated alkyl having 1-4 carbon atoms, or cycloalkyl having 3-4 carbon atoms, |
| | but with the exception of those compounds in which side by side $R^1$ is OH or SH, $R^2$ is H, $R^3$ is $Y\text{-}R^4$, X is O or S, Y is O and $R^4$ is alkyl having 1-4 carbon atoms. |

3. A process as claimed in claim 1 or 2, wherein compounds are prepared in which $R^4$ is partially fluorinated alkyl having 1-4 carbon atoms, or cycloalkyl having 3-4 carbon atoms.

4. A process as claimed in claim 1 or 2, wherein compounds are prepared in which Y is S, SO or $SO_2$.

5. A process as claimed in one or more of claims 1 to 4, wherein compounds are prepared in which $R^1$ is H, halogen, SH or $NH_2$.

6. A process as claimed in one or more of claims 1 to 4, wherein compounds are prepared in which $R^1$ is H, OH or $NH_2$, $R^2$ is acyl, in particular acetyl and X is O or S.

7. A process as claimed in one or more of claims 1 to 5, wherein compounds are prepared in which, in $R^4$ to $R^6$, the acyclic hydrocarbon radical has 3 to 5 carbon atoms and the cycloaliphatic radical has 4 to 6 and, in particular, 5 to 6 carbon atoms, and any halogen contained in $R^1$ is chlorine or bromine, and any halogen contained in $R^3$ is fluorine or chlorine.

8. A process for preparing a pharmaceutical composition which is suitable for treating diseases caused by viruses, which comprises incorporating as active substance a compound of the formula 1 which is prepared by the process as claimed in one or more of claims 1 to 7, in combination with a pharmaceutically acceptable vehicle in such a way that a composition suitable for administration is obtained.

9. A process for preparing medicament formulations for treating diseases caused by viruses, which comprises formulating a compound 1 which has been obtained by the process as claimed in one or more of claims 1 to 7 with the usual excipients and auxiliaries.

10. The process as claimed in claim 1, wherein the following compounds are prepared: 9-(1-isopropoxymethyl-2-acetoxyethoxymethyl)-guanine, 2-amino-9-(1-isopropoxymethyl-2-acetoxyethoxymethyl)-purine, 2-amino-6-isopropoxy-9-(1-isopropoxymethyl-2-acetoxyethoxy-methyl)-purine, 2-amino-9-[1,3-bis-(isopropoxy)-propyl-2-oxymethyl]-purine and 2-amino-6-isopropoxy-9-[1,3-bis-(isopropoxy)-propyl-2-oxymethyl]-purine.

11. The process as claimed in claim 1, wherein 9-(1-isopropoxymethyl-2-hydroxyethoxymethyl)-guanine is prepared.

**Revendications**

1. Composés de formule 1

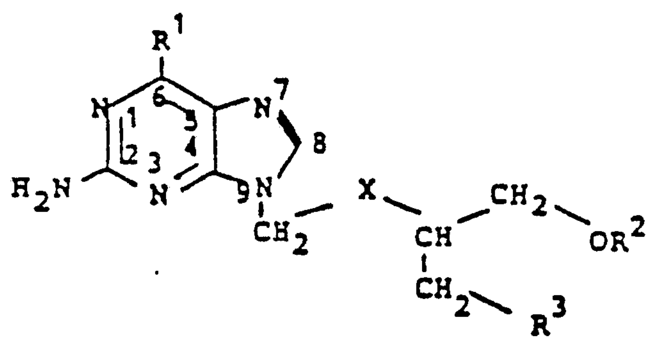

dans laquelle

$R^1$ est H, halogène, OH, SH, azido, $NH_2$ ou Z-$R^5$, et Z est O, S, SO, $SO_2$ ou NH, et $R^5$ représente un reste d'hydrocarbure aliphatique acyclique en $C_{1-6}$, qui est a) non-subtitué ou b) modifié par une au moins des caractéristiques suivantes, à savoir qu'il renferme un groupe alcoxy en $C_{1-3}$ ou une double liaison, ou bien qu'il est au moins partiellement fluoré, ou encore qu'il peut être substitué par un cycloalkyle en $C_{3-6}$ ou phénylalkyle, lié à Z directement ou par l'intermédiaire d'un groupe alkylène de 1 à 3 atomes de carbone, ou phényle, le cycle phényle pouvant être substitué chaque fois par au plus deux, de préférence au plus un susbtituant choisi parmi halogène, trifluorométhyle, alcoxy et alkylthio en $C_{1-3}$ chacun,

$R^2$ est H ou bien prend les définitions de $R^5$ ou est un groupe acyle CO-$R^6$, $R^6$ étant alkyle en $C_{1-6}$, benzyle ou phényle,

$R^3$ est halogène, azido, amino non-substitué ou le reste Y-$R^4$,

$R^4$ ayant les mêmes définitions que $R^5$ et

Y, ainsi que X, sont O, S, SO ou $SO_2$, X et Y pouvant être identiques ou différents, à l'exclusion de la 9-(1,3-bis-(isopropoxy)-propyl-2-oxyméthyl)-guanine et des composés similaires, dans lequels, en même temps, $R^1$ est OH ou SH, $R^2$ est H, $R^3$ est Y-$R^4$, X est O ou S, Y est O et $R^4$ est alkyle en $C_{1-6}$, ou bien dans lesquels, en même temps, $R^1$ est OH ou $NH_2$, $R^2$ est benzyle, R3 est Y-$R^4$ avec Y=O et $R^4$ =benzyle, et X est O.

2. Composés selon la revendication 1, caractérisés en ce que

$R^1$ est H, halogène, OH, SH ou $NH_2$,

$R^2$ est H ou acyle,
$R^3$ est halogène ou Y-$R^4$,
X et Y sont O, S, SO ou $SO_2$, et sont identiques ou différents,
et
R4 représente un reste d'hydrocarbure aliphatique acyclique en $C_{1-6}$, un alkyle en $C_{1-4}$ au moins partiellement fluoré, ou un cycloalkyle en $C_{3-4}$, à l'exception toutefois des composés dans lequels, en même temps, $R^1$ est OH ou SH, $R^2$ est H, $R^3$ est Y-$R^4$, X est O ou S, Y est O et $R^4$ est alkyle en $C_{1-6}$.

**3.** Composés selon l'une des revendications 1 ou 2, caractérisés en ce que $R^4$ représente un alkyle en $C_{1-4}$ partiellement fluoré ou un cycloalkyle en $C_{3-4}$.

**4.** Composés selon l'une des revendications 1 ou 2, caractérisés en ce que Y est S, SO ou $SO_2$.

**5.** Composés selon une ou plusieurs des revendications 1 à 4, caractérisés en ce que $R^1$ est H, halogène, SH ou $NH_2$.

**6.** Composés selon une ou plusieurs des revendications 1 à 4, caractérisés en ce que $R^1$ est H, OH ou $NH_2$, $R^2$ est acyle, en particulier acétyle, et X est O ou S.

**7.** Composés selon une ou plusieurs des revendications 1 à 5, caractérisés en ce que, dans les restes $R^4$ à $R^6$, le reste d'hydrocarbure acyclique comporte de 3 à 5 atomes de carbone et le reste cycloaliphatique de 4 à 6 atomes de carbone et en particulier de 5 à 6 atomes de carbone, et que $R^1$ contient du chlore ou du brome et $R^3$ du fluor ou du chlore, comme halogène.

**8.** Médicaments, caractérisés en ce qu'ils renferment une proportion active d'au moins un composé selon une ou plusieurs des revendications 1 à 7.

**9.** Procédé de préparation de composés selon une ou plusieurs des revendications 1 à 7, caractérisé en ce que l'on fait réagir, dans les conditions usuelles,
A) pour la préparation de composés dans lesquels $R^1$ est hydroxy, amino ou hydrogène, une 2,6,9-tris-(trialkylsilyl)- ou 2,9-bis-(trialkylsilyl)-2-aminopurine répondant à une des formules 8, 11 et 12

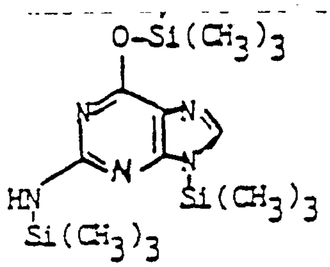
8

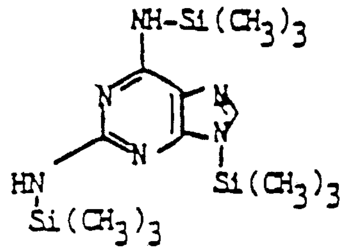
11

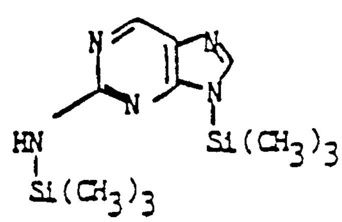
12

avec des composés halogénés, en particulier chlorés, de formule

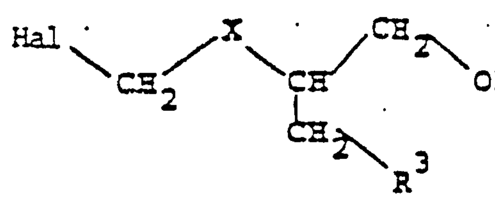
13

dans laquelle $R^2$ est acyle et X et $R^3$ ont les définitions données dans les revendications 1 à 7, et on coupe le groupe protecteur trialkylsilyle, ou bien
B) pour la préparation de composés dans lesquels $R^1$ est hydroxy, mercapto, amino ou hydrogène, les composés di- ou triacylés de formules 9, 10, 6a ou 7a

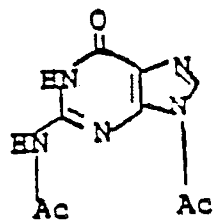

9

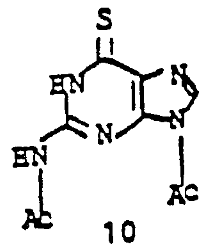

10

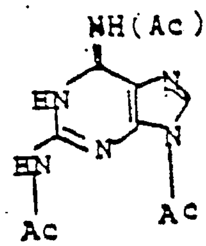

6a

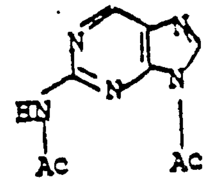

7a

avec des composés acétoxylés réactifs de formule 14

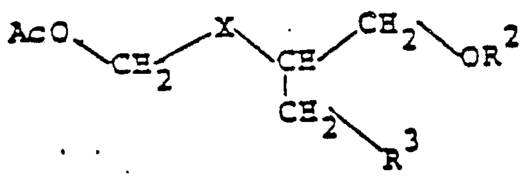

14

dans laquelle $R^2$ est acyle et X et et $R^3$ ont les définitions données dans les revendications 1 a 7, ou

C) pour la préparation de composés dans lesquels $R^1$ est halogène, des composés de formule 5

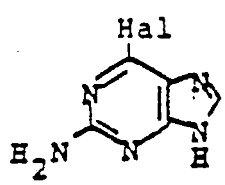

5

avec des composés halogénés actifs de la formule 13 indiquée sous A, et on isole le composé 18 ainsi obtenu ou bien on le transforme, par saponification, en composés dans lesquels $R^1$ est hydroxy, ou, par réaction avec du sulfure d'hydrogène, en composés dans lesquels $R^1$ est mercapto, ou, par réaction avec des azotures alcalins, en composés dans lesquels $R^1$ est azido, ou, par réaction avec l'ammoniac, en composés dans lesquels $R^1$ est amino, ou, par déshydrohalogénation catalytique, en composés dans lesquels $R^1$ est hydrogène, ou, par réaction avec des alcools ou phénols, des mercaptans ou thiophénols ou des amines ou anilines, en composés dans lesquels $R^1$ est ZR5, ou bien

D) pour la préparation de composés dans lesquels $R^2$ est hydrogène, on saponifie un composé préparé selon un des procédés A à C.

10. Utilisation des composés selon une ou plusieurs des revendications 1 à 7 ou de 9-(1,3-bis-(isopropoxy)-propyl-2-oxyméthyl)-guanine pour la fabrication de médicaments pour le traitement de maladies virales.

11. Composé selon la revendication 1, caractérisé en ce qu'il est la 9-(1-isopropoxyméthyl-2-acétoxyéthoxyméthyl)-guanine, la 2-amino-9-(1-isopropoxyméthyl-2-acétoxyéthoxyméthyl)-purine, la 2-amino-6-isopropoxy-9-(1-isopropoxyméthyl-2-acétoxyéthoxyméthyl)-purine, la 2-amino-9-[1,3-bis-(isopropoxy)-propyl-2-oxyméthyl)-purine ou la 2-amino-6-isopropoxy-9-[1,3-bis-(isopropoxy)propyl-2-oxyméthyl]-purine.

**12.** La 9-(1-isopropoxyméthyl-2-hydroxyéthoxyméthyl)-guanine.

Revendication pour l'Etat contractant suivant: AT

**1.** Procédé de préparation de composés de formule 1

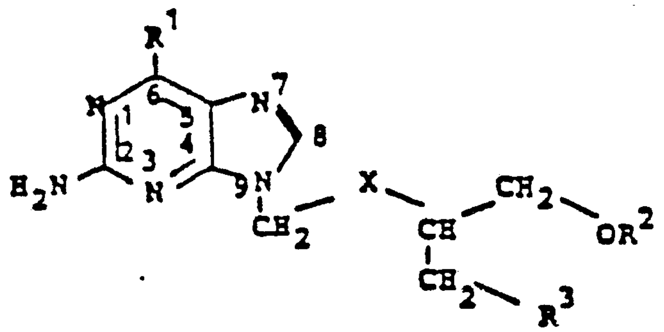

dans laquelle

$R^1$ est H, halogène, OH, SH, azido, $NH_2$ ou Z-$R^5$, et Z est O, S, SO, $SO_2$ ou NH, et $R^5$ représente un reste d'hydrocarbure aliphatique acyclique en $C_{1-6}$, qui est a) non-subtitué ou b) modifié par une au moins des caractéristiques suivantes, à savoir qu'il renferme un groupe alcoxy en $C_{1-3}$ ou une double liaison, ou bien qu'il est au moins partiellement fluoré, ou encore qu'il peut être substitué par un cycloalkyle en $C_{3-6}$ ou phénylalkyle, lié à Z directement ou par l'intermédiaire d'un groupe alkylène de 1 à 3 atomes de carbone, ou phényle, le cycle phényle pouvant être substitué chaque fois par au plus deux, de préférence au plus un susbtituant choisi parmi halogène, trifluorométhyle, alcoxy et alkylthio en $C_{1-3}$ chacun,

$R^2$ est H ou bien prend les définitions de $R^5$ ou est un groupe acyle CO-$R^6$, $R^6$ étant alkyle en $C_{1-6}$, benzyle ou phényle,

$R^3$ est halogène, azido, amino non-substitué ou le reste Y-$R^4$,

$R^4$ ayant les mêmes définitions que $R^5$ et

Y, ainsi que X, sont O, S, SO ou $SO_2$, X et Y pouvant être identiques ou différents,

à l'exclusion de la 9-(1,3-bis-(isopropoxy)-propyl-2-oxyméthyl)-guanine et des composés similaires, dans lequels, en même temps, $R^1$ est OH ou SH, $R^2$ est H, $R^3$ est Y-$R^4$, X est O ou S, Y est O et $R^4$ est alkyle en $C_{1-6}$, ou bien dans lesquels, en même temps, $R^1$ est OH ou $NH_2$, $R^2$ est benzyle, $R^3$ est Y-$R^4$ avec Y = O et $R^4$ = benzyle, et X est O,

caractérisé en ce que l'on fait réagir, dans les conditions usuelles,

A) pour la préparation de composés dans lesquels $R^1$ est hydroxy, amino ou hydrogène, une 2,6,9-tris-(trialkylsilyl)- ou 2,9-bis-(trialkylsilyl)-2-aminopurine répondant à une des formules 8 11 or 12

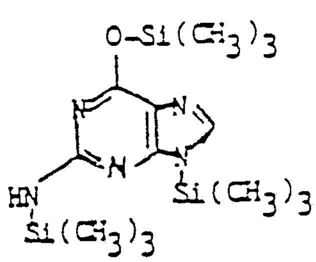

8

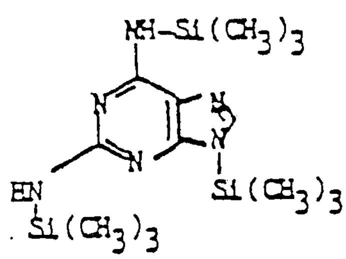

11

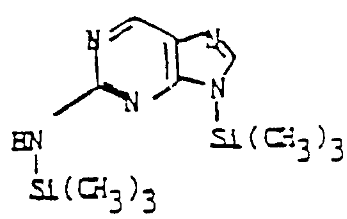

12

avec des composés halogénés, en particulier chlorés, de formule

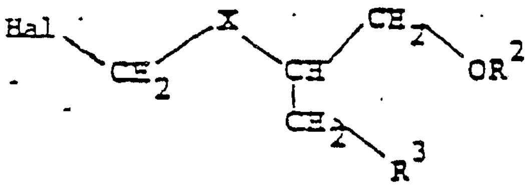

13

dans laquelle $R^2$ est acyle et X et $R^3$ ont les définitions données ci-dessus, et on coupe le groupe protecteur trialkylsilyle, ou bien

B) pour la préparation de composés dans lesquels $R^1$ est hydroxy, mercapto, amino ou hydrogène, les composés di- ou triacylés de formules 9, 10, 6a ou 7a

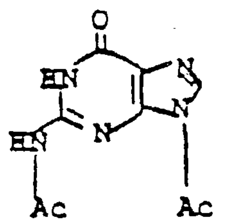

9

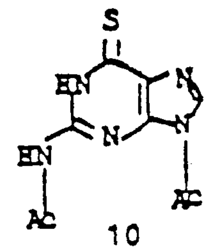

10

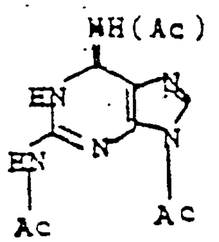

6a

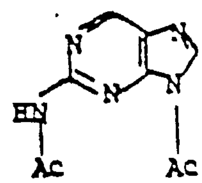

7a

avec des composés acétoxylés réactifs de formule 14

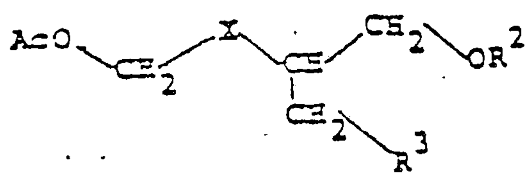

14

dans laquelle $R^2$ est acyle et X et et $R^3$ ont les définitions données ci-dessus, ou

C) pour la préparation de composés dans lesquels $R^1$ est halogène, des composés de formule 5

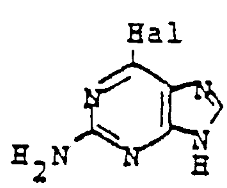

5

avec des composés halogénés actifs de la formule 13 indiquée sous A, et on isole le composé 18 ainsi obtenu ou bien on le transforme, par saponification, en composés dans lesquels $R^1$ est hydroxy, ou, par réaction avec du sulfure d'hydrogène, en composés dans lesquels $R^1$ est mercapto, ou, par réaction avec des azotures alcalins, en composés dans lesquels $R^1$ est azido, ou, par réaction avec l'ammoniac, en composés dans lesquels $R^1$ est amino, ou, par déshydrohalogénation catalytique, en composés dans lesquels $R^1$ est hydrogène, ou, par réaction avec des alcools ou

phénols, des mercaptans ou thiophénols ou des amines ou anilines, en composés dans lesquels $R^1$ est ZR5, ou bien
D) pour la préparation de composés dans lesquels $R^2$ est hydrogène, on saponifie un composé préparé selon un des procédés A à C.

2. Procédé selon la revendication 1, caractérisé en ce que l'on prépare des composés dans lesquels
$R^1$ est H, halogène, OH, SH ou $NH_2$,
$R^2$ est H ou acyle,
$R^3$ est halogène ou Y-$R^4$,
X et Y sont O, S, SO ou $SO_2$, et sont identiques ou différents,
et
$R^4$ représente un reste d'hydrocarbure aliphatique acyclique en $C_{1-6}$, un alkyle en $C_{1-4}$ au moins partiellement fluoré, ou un cycloalkyle en $C_{3-4}$, à l'exception toutefois des composés dans lequels, en même temps, $R^1$ est OH ou SH, $R^2$ est H, $R^3$ est Y-$R^4$, X est O ou S, Y est O et $R^4$ est alkyle en $C_{1-6}$.

3. Procédé selon l'une des revendications 1 ou 2, caractérisé en ce que l'on prépare des composés dans lesquels $R^4$ représente un alkyle en $C_{1-4}$ partiellement fluoré ou un cycloalkyle en $C_{3-4}$.

4. Procédé selon l'une des revendications 1 ou 2, caractérisé en ce que l'on prépare des composés dans lesquels Y est S, SO ou $SO_2$.

5. Procédé selon une ou plusieurs des revendications 1 à 4, caractérisé en ce que l'on prépare des composés dans lesquels $R^1$ est H, halogène, SH ou $NH_2$.

6. Procédé selon une ou plusieurs des revendications 1 à 4, caractérisé en ce que l'on prépare des composés dans lesquels $R^1$ est H, OH ou $NH_2$, $R^2$ est acyle, en particulier acétyle, et X est O ou S.

7. Procédé selon une ou plusieurs des revendications 1 à 5, caractérisé en ce que l'on prépare des composés dans lesquels, dans les restes $R^4$ à $R^6$, le reste d'hydrocarbure acyclique comporte de 3 à 5 atomes de carbone et le reste cycloaliphatique de 4 à 6 atomes de carbone et en particulier de 5 à 6 atomes de carbone, et que $R^1$ contient du chlore ou du brome et $R^3$ du fluor ou du chlore, comme halogène.

8. Procédé pour la fabrication d'une composition pharmaceutique pour le traitement des maladies virales, caractérisé en ce que l'on incorpore, comme substance active, un composé de formule 1, préparé d'après un procédé conforme à une ou plusieurs des revendications 1 à 7, en association avec un véhicule pharmaceutiquement acceptable, de manière à obtenir une composition apte à être administrée.

9. Procédé pour la fabrication de formulations médicamenteuses pour le traitement de maladies virales, caractérisé en ce que l'on formule en médicaments, a l'aide des véhicules et adjuvants usuels, un composé de formule 1, qui a été obtenu par un procédé conforme à une ou plusieurs des revendications 1 à 7.

10. Procédé selon la revendication 1, caractérisé en ce que l'on prépare les composés suivants:
9-(1-isopropoxyméthyl-2-acétoxyéthoxyméthyl)-guanine,
2-amino-9-(1-isopropoxyméthyl-2-acétoxyéthoxyméthyl)-purine,
2-amino-6-isopropoxy-9-(1-isopropoxyméthyl-2-acétoxyéthoxyméthyl)-purine,
2-amino-9-[1,3-bis-(isopropoxy)propyl-2-oxyméthyl)-purine et 2-amino-6-isopropoxy-9-[1,3-bis-(isopropoxy)propyl-2-oxyméthyl]-purine.

11. Procédé selon la revendication 1, caractérisé en ce que l'on prépare la 9-(1-isopropoxyméthyl-2-hydroxyéthoxyméthyl)-guanine.

FIG.1

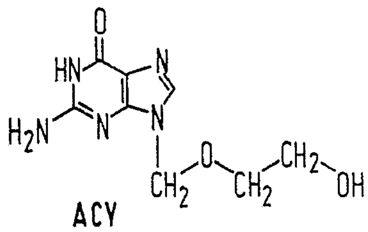

ACY

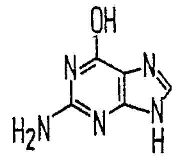

2'

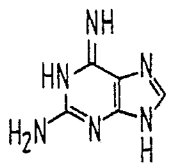

3'

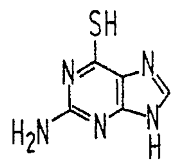

6'

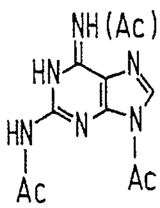

6a

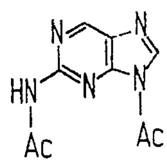

7a

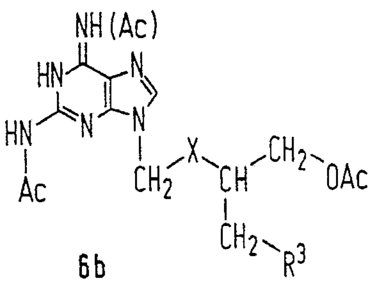

6b

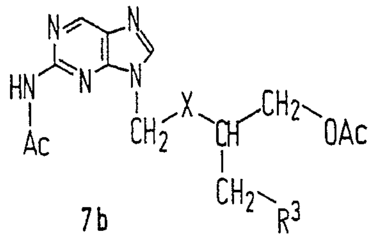

7b

FIG.2 / Teil 1

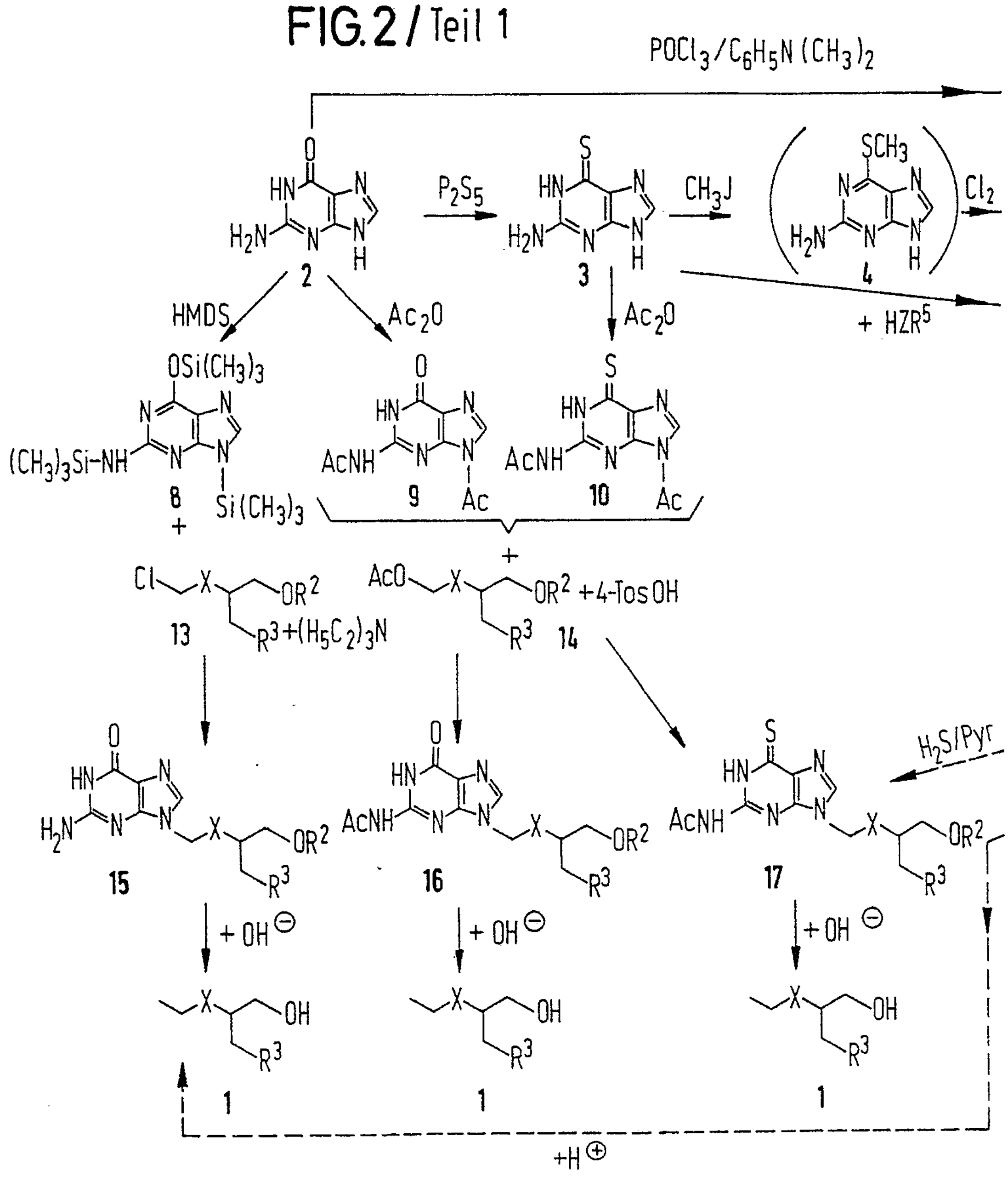

$POCl_3/C_6H_5N(CH_3)_2$
$P_2S_5$
$CH_3J$
$Cl_2$
2
3
4
HMDS
$Ac_2O$
$Ac_2O$
+ $HZR^5$
8
9
10
+
+
13
$+(H_5C_2)_3N$
+4-TosOH
14
$H_2S/Pyr$
15
16
17
$+OH^{\ominus}$
$+OH^{\ominus}$
$+OH^{\ominus}$
1
1
1
$+H^{\oplus}$

FIG.2/Teil 2

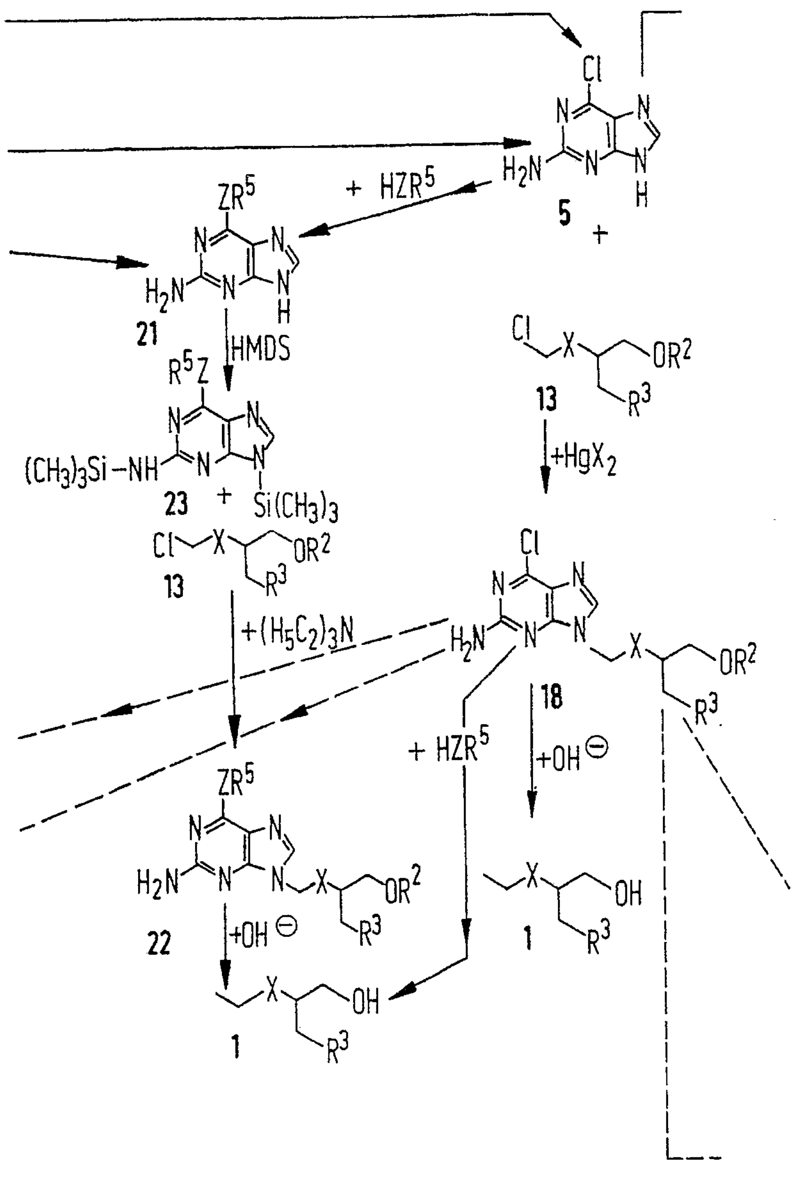

FIG. 2/Teil 3

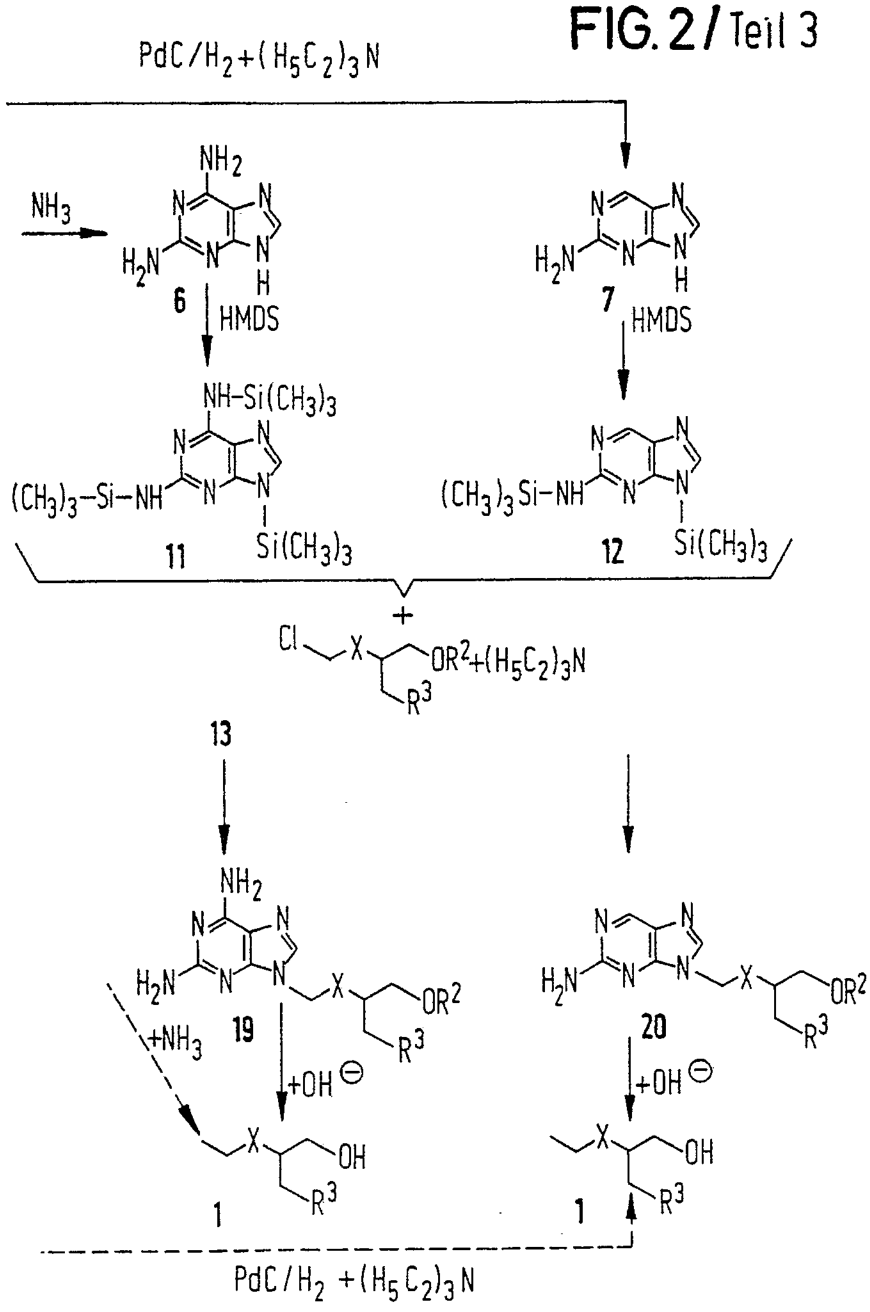


6
7
11
12
13
19
20
1
1